(19) Europäisches Patentamt
European Patent Office
Office européen des brevets

(11) Publication number: **0 207 600 B1**

(12) # EUROPEAN PATENT SPECIFICATION

(45) Date of publication of patent specification: **29.01.92**

(51) Int. Cl.5: **C07C 217/60**, C07C 219/24, C07C 233/10, C07C 255/31, C07C 255/32, C07C 233/11, C07C 205/18, C07C 251/00, A61K 31/13, A61K 31/215

(21) Application number: **86303502.8**

(22) Date of filing: **08.05.86**

(54) **Phenoxyethylamine derivatives.**

(30) Priority: **09.05.85 US 732478**

(43) Date of publication of application: **07.01.87 Bulletin 87/02**

(45) Publication of the grant of the patent: **29.01.92 Bulletin 92/05**

(84) Designated Contracting States: **AT BE CH DE FR IT LI LU NL SE**

(56) References cited: **EP-A- 0 112 669**

(73) Proprietor: **AMERICAN HOME PRODUCTS CORPORATION**
**685, Third Avenue**
**New York, New York 10017(US)**

(72) Inventor: **Husbands, George Edward Morris**
**258 Dayleview Road Berwyn**
**Chester Pennsylvania(US)**

(74) Representative: **Wileman, David Francis et al**
**c/o John Wyeth and Brother Limited Huntercombe Lane South**
**Taplow Maidenhead Berkshire SL6 OPH(GB)**

EP 0 207 600 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a group of substituted phenoxyethylamine derivatives which are central nervous system antidepressants, to processes for preparing them and to pharmaceutical compositions containing them. EP Publication No. 112,669 describes certain phenethylamine derivatives and intermediates therefor, which derivatives possess central nervous system depressant activity.

This invention provides a compound having the following structural formula:

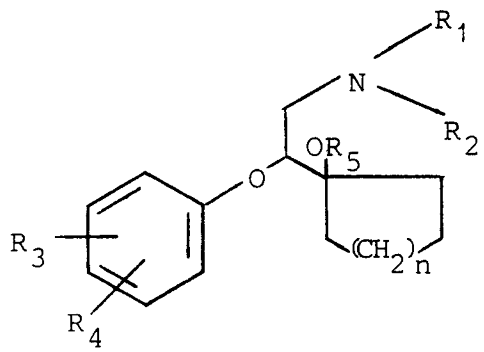

(I)

in which $R_1$ is hydrogen or alkyl of 1 to 6 carbon atoms; $R_2$ is alkyl of 1 to 6 carbon atoms; $R_3$ and $R_4$ are independently hydrogen, hydroxyl, alkyl of 1 to 6 carbon atoms, alkoxy of 1 to 6 carbon atoms, alkanoyloxy of 2 to 7 carbon atoms, halo or trifluoromethyl; $R_5$ is hydrogen, alkyl of 1 to 6 carbon atoms or alkanoyl of 2 to 7 carbon atoms; and n is one of the integers 0,1,2 or 3; or a pharmaceutically acceptable salt thereof.

The compounds in which $R_5$ is alkanoyl of 2 to 7 carbon atoms are less potent than those where $R_5$ is hydrogen. However, in long term therapy the acyloxy derivatives will act as pro drugs as the acyl group may be removed in vivo either via acid hydrolysis in the stomach or enzymatically.

The pharmaceutically acceptable acid addition salts of the basic compounds of this invention are formed conventionally by reaction of the free base with an equivalent amount of any acid which forms a non-toxic salt. Illustrative acids are either inorganic or organic, including hydrochloric, hydrobromic, fumaric, maleic, succinic, sulfuric, phosphoric, tartaric, acetic, citric, oxalic and similar acids. For parenteral administration, the use of water soluble salts is preferred, although either the free base of the pharmaceutically acceptable salts are applicable for oral or parenteral administration of the antidepressant agents of this invention. The halo substituent representing $R_3$ or $R_4$ is intended to include the chloro, bromo, iodo or fluoro substituents.

Preferred values for $R_1$ are alkyl of 1 to 3 carbon atoms. Preferably $R_2$ is alkyl of 1 to 3 carbon atoms. Examples of $R_3$ are hydrogen, alkoxy of 1 to 3 carbon atoms, chloro, bromo and $CF_3$. $R_4$ is preferably alkyl of 1 to 3 carbon atoms, alkoxy of 1 to 3 carbon atoms, chloro, bromo or $CF_3$. Preferably n is 1, 2 or 3. $R_5$ is preferably hydrogen.

Accordingly preferred compounds are those of the formula:

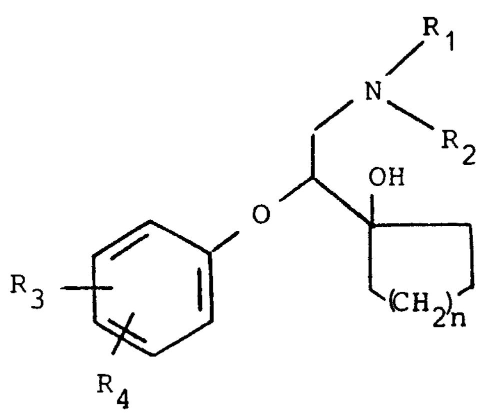

(Ia)

in which

$R_1$ is alkyl of 1 to 3 carbon atoms;
$R_2$ is alkyl of 1 to 3 carbon atoms;
$R_3$ is hydrogen, alkoxy of 1 to 3 carbon atoms, chloro, bromo or trifluoromethyl;
$R_4$ is alkyl of 1 to 3 carbon atoms, alkoxy of 1 to 3 carbon atoms, chloro, bromo or trifluoromethyl;
and n is one of the integers 1, 2 or 3;
or a pharmaceutically acceptable salt thereof.

The most preferred compounds are those in which $R_3$ and $R_4$ are in meta or para positions.

This invention provides processes for preparing the compounds of the invention.

The compounds of this invention are prepared by reaction of a cycloalkanone with an appropriately substituted phenoxyacetamide anion following the procedure of Sauvetre et al., Tetrahedron, 34,2135 (1978), followed by reduction of the amide with for example an aluminium hydride (e.g. lithium aluminium hydride) or a borane reducing agent to give the corresponding amine. This method permits one to readily vary the values $R_1$ and $R_2$ in the initial reactant.

The intermediate amide represents an additional aspect of this invention and is depicted by the following structural formula:

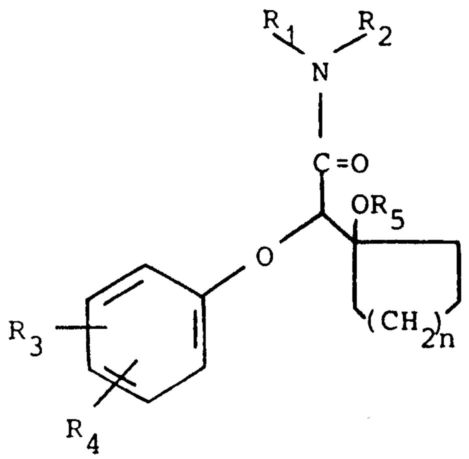

(II)

in which n, $R_1$, $R_2$, $R_3$ and $R_4$ are as defined in connection with formula I, $R_2$ also represents hydrogen, and $R_5$ is hydrogen or alkyl of 1 to 6 carbon atoms.

Preferred values of $R_1$, $R_2$, $R_3$, $R_4$ and $R_5$ for intermediates of formula II are the same as for the final products. When $R_5$ is alkyl it is introduced prior to reduction of the carbonyl group by conventional O-alkylation.

During the course of the synthesis of the compounds of this invention any hydroxy group represented by $-OR_5$, $F_3$ or $R_4$ may be in the free form or in the form of hydroxy protected by a removable protecting group, except of course, that the hydroxy group is not protected in any case where it is intended to participate in a reaction. The protected form is recommended where the hydroxy group may otherwise undergo an undesired reaction. Examples of protecting groups for hydroxy are given in Protective Groups in Organic Chemistry edited by J.F.W.McOmie, Chapters 3 and 4 (pages 95-182), published by Plenum Press (1973), and protective Groups in Organic Chemistry by T.W.Greene, Chapters 2 and 3 (pages 10 to 113) published by John Wiley and Sons (1981). The protecting group may be removed at a suitable later stage in the synthesis.

The compounds of this invention may also be produced by reaction of an appropriate cycloalkanone with chloroacetonitrile to obtain a cycloalkylacetonitrile-$\alpha,\beta$-epoxide which reacts with an appropriately substituted alkali metal phenate to obtain the correspondingly substituted $\alpha$-(1-hydroxycycloalkyl)-phenoxyacetonitrile. Reduction of the nitrile with a boran hydride reducing agent (diborane) yields the primary amine which is conventionally N-alkylated to afford the final products.

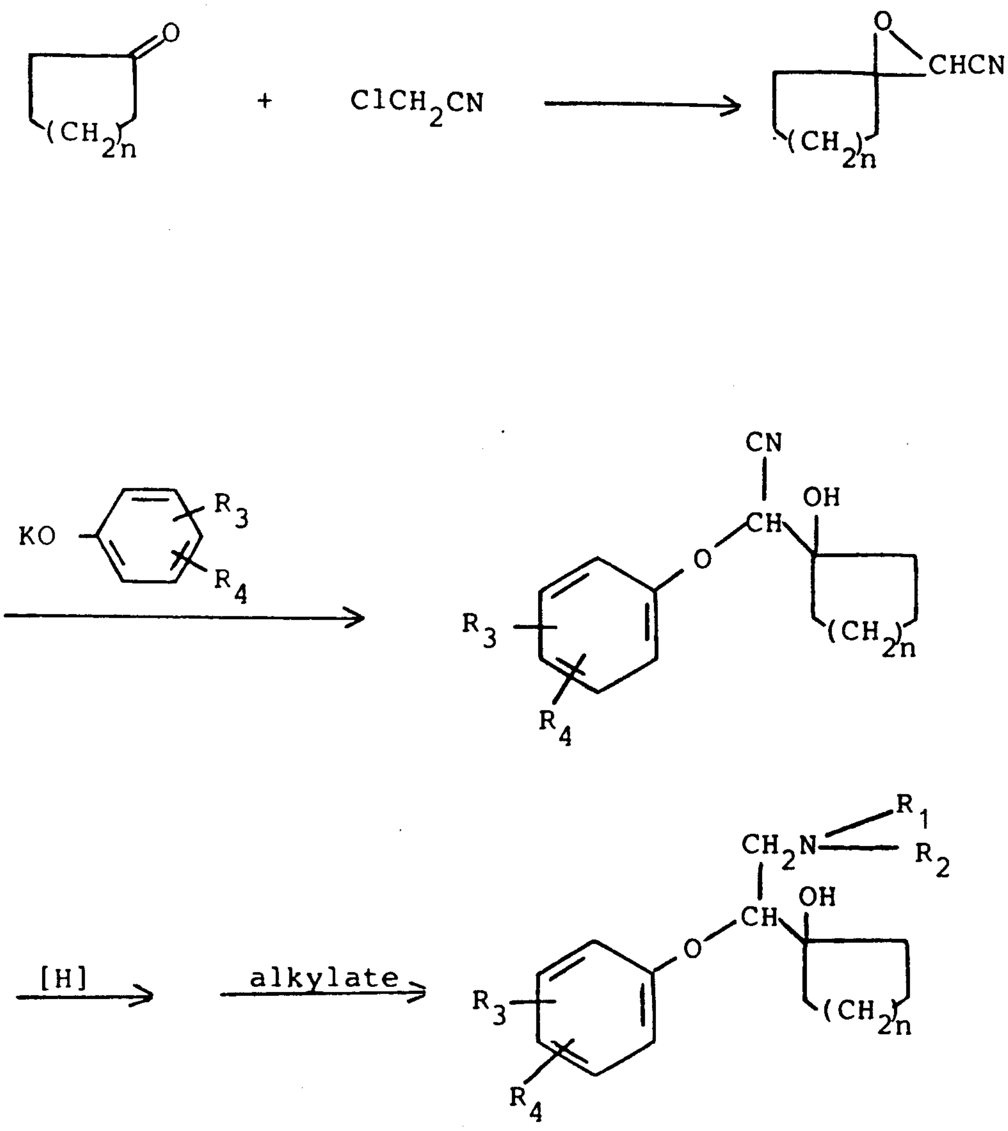


Throughout this procedure, any hydroxyl group represented by $-OR_5$, $R_3$ or $R_4$ may be in the free form or protected in the same manner as with the amide reactants discussed, supra.

More indirect routes for synthesis of the antidepressant compounds of this invention involve the reaction of a cycloalkanone with an anion of an appropriately substituted phenoxyacetic acid, salt, ester or aldehyde

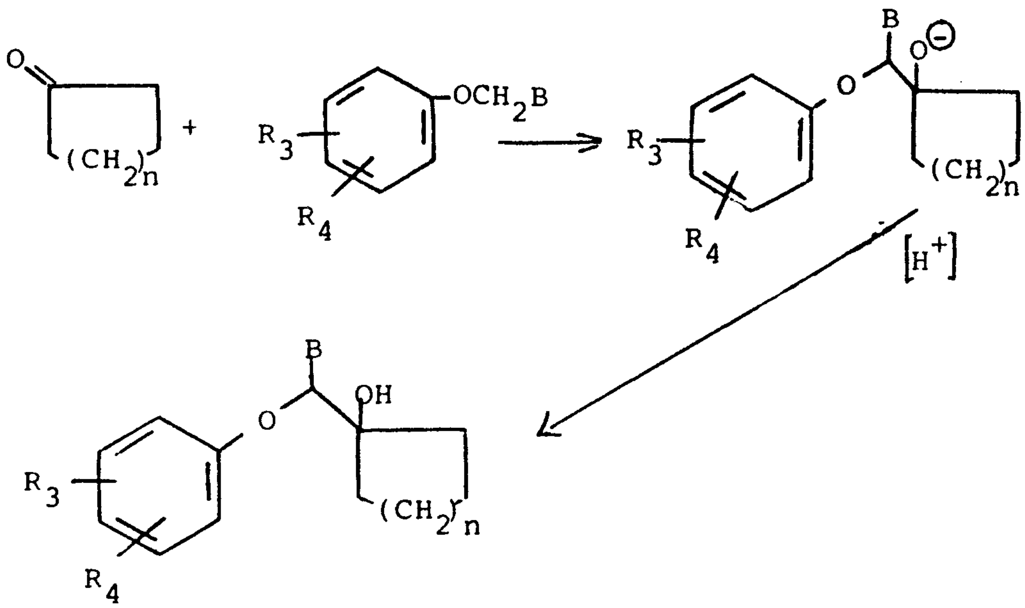


where B represents a carboxyl group or its salt or ester or a -CHO functional group.

The carboxylic acid group may be converted to an acid halide, active ester or anhydride and directly reacted with the desired amine to yield, after reduction of the resulting amide, the end products of this invention. Also, the carboxylic acid group may be reduced with diisobutyl aluminium hydride or lithium aluminium hydride to obtain the corresponding aldehyde. The ester is readily converted to the aldehyde with diisobutyl aluminium or to the alcohol with lithium aluminium hydride. The aldehyde may be condensed with hydroxylamine to afford the oxime -CH = NOH; with ammonium or a primary amine to afford an imine -CH = $NR_1$ or with a primary or secondary amine to afford

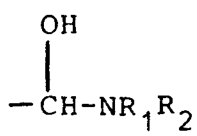

An alcohol $-CH_2OH$, produced through reduction of the carboxylic acid, salt or aldehyde may be converted to the corresponding nitro derivative by producing an organic sulfonate (mesyl ester) or halide followed by displacement with an inorganic nitrite. Reduction of these intermediates yields the primary amine intermediates or the secondary or tertiary amine end products of this invention. The alcohol may also be converted to mesylates or tosylates, reacted with KCN to afford the nitrile, converted to the amide and subjected to a Hoffman rearrangement with bromine or chlorine and an alkali metal hydroxide.

Additional routes to the desired products include the reaction of ammonia or $HNR_1R_2$ with

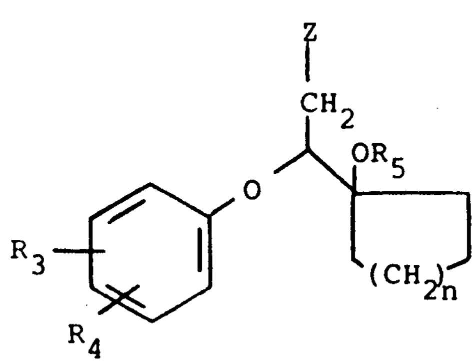

where Z is a leaving group such as halogen, obtained by displacement of a hydroxyl group, or an organo sulfonyloxy (mesyl, tosyl and the like) group, obtained by acylation of a hydroxyl group, under conventional conditions. If desired, the amine reactant may be initially blocked with a relatively labile acyl group such as trifluoroacetyl to provide a reactant of the formula

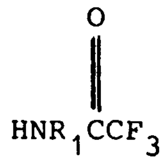

prior to reaction with the alkylating reactant employing KOH and a very polar solvent such as dimethylsulfoxide, to provide a tertiary amide from which the acyl group may be readily removed to prepare the compound for non-symmetrical N-alkylation to insert $R_2$. Rather than N-alkylate, one may acylate or react the secondary amine with an aldehyde and subsequently reduce the amide or Schiff base. Similarly, reaction of the amine with an alkylchloroformate affords, upon reduction, an N-methylated amine.
$LiAlH_4$ is a good reducing agent for these processes.

Reductive amination of the aldehyde

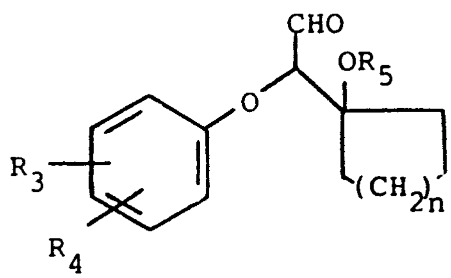

with ammonia, a primary amine or a secondary amine (Leuckart reaction) also yields the desired end products.

Similarly, during the course of the synthesis of the end compounds of the invention by means of those more indirect processes identified above, any hydroxy group represented by $-OR_5$, $R_3$ or $R_4$ may be in the free base form or in the form of hydroxy protected by a removable protecting group, as indicated, supra.

Accordingly this invention provides a process for preparing a compound of formula I or a pharmaceutically acceptable salt thereof which comprises:

(i) reducing a compound of formula A:

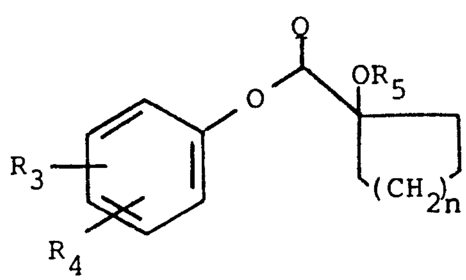

(A)

where Q represents $-CONR_1R_2$; $-CH=NR_2$;
$-CH(OH)NR_1R_2$; $-CH_2NR_1COR_9$; $-CH_2N=CY_1Y_2$; $-CH_2NR_1CH(OH)R_8$,

$R_9$ represents hydrogen, alkoxy or $C_{1-5}$ alkyl;

$Y_1$ and $Y_2$ are each selected from hydrogen and alkyl such that the group $=CY_1Y_2$ contains 1 to 6 carbon atoms

$R_8$ is $C_1$-$C_5$ alkyl

and n, $R_3$, $R_4$ and $R_5$ are as hereinbefore defined,

or (2) reacting a compound having formula B

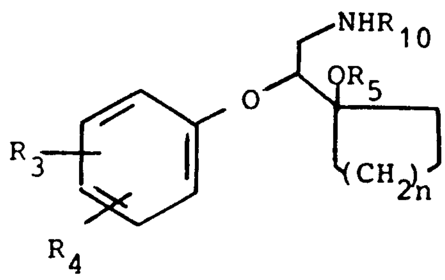

(B)

where $R_{10}$ is $R_1$ or a readily splittable substituent, and A, $R_1$, $R_5$, $R_6$ and $R_7$ are as defined under formula I with an alkylating agent to introduce one or two $C_1$-$C_6$ alkyl groups (if $R_{10}$ is H) or one such alkyl group and, if necessary, splitting off the said readily splittable substituent;
or (b) reacting a compound of formula B as defined above wherein $R_{10}$ is $R_1$ with a carbonyl compound of formula $Y_1Y_2CO$ [where $Y_1$ and $Y_2$ are as defined above] in the presence of a reducing agent; or
(3)(a) reacting of a compound of formula $HNR_2R_{10}$ (where $R_1$, $R_2$ and $R_{10}$ are as defined above) with a compound having the formula C

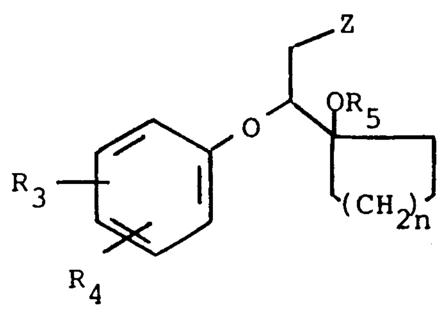

(C)

(where Z is a leaving group and $R_3$, $R_4$ and $R_5$ are as defined under formula I and, if necessary, splitting off the substituent $R_{10}$;
or (b) reacting a compound of formula $HNR_1R_2$ (where $R_1$ and $R_2$ are as defined above) with an aldehyde of formula D

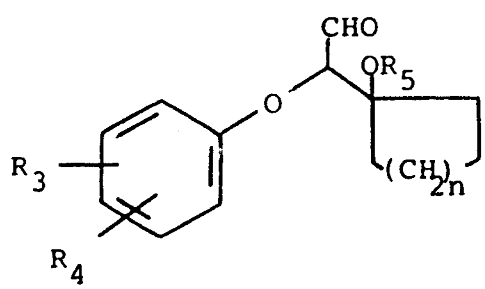

(D)

(where $R_3$,$R_4$,$R_5$ and n are as defined under formula I in the presence of a reducing agent; or
(4) reacting a compound of formula I wherein $R_5$ is hydrogen with a reactive derivative of a $C_{2-7}$ alkanoic acid to introduce $C_{2-7}$ alkanoyl as $R_5$; or
(5) forming a compound of formula I or salt thereof by removal of a protecting group to give hydroxy as $R_3$ and/or $R_4$ and/or $OR_5$, or
(6) reacting a compound of formula I in free base form with an acid to form a pharmaceutically acceptable salt thereof or reacting a salt of a compound of formula I with a base to give the compound of formula I in free base form.

The end products contain one asymmetric centre. Individual stereoisomeric forms may be obtained or

separated by standard procedures. For instance, separation of the mixture may be carried out by neutralization with a suitable optically active compound to form salts which can be separated.

The antidepressant activity of the compounds of this invention was established by demonstrating that they inhibit synaptosomal uptake of norepinephrine ($^3$H-NE) and serotonin ($^{14}$C-5-HT) following the test procedure of Wood et al., J.Neurochem., 37, 795-797 (1981).

The results of these procedures affirmed the antidepressant activity of the compounds of this invention agreement with the most widely accepted theory of antidepressant activity and in correlation of activity with known tricyclic anti depressants. In at least two instances, namely, with the 2,4-dichloro product of Example 2, and 4-chloro product in Example 1, the undesirable attribute of classical antidepressants observed as an anticholinergic property which is reflected by the inhibition of binding of the muscarinic receptor ligand, 3H-quinuclidinyl benzilate (QNB) is missing.

Inhibition of synaptosomal NE and 5-HT uptake:

Results of the inhibition of NE and 5-HT synaptosomal uptake, expressed as the inhibitory concentration at which the rate of uptake was reduced to 50 percent ($IC_{50}$), are presented in the table below, where they are compared with the values for imipramine, DMI, amitriptyline and fluoxetine:

| Compound | NE | 5-HT |
|---|---|---|
| Imipramine | 0.26 | 0.12 |
| DMI | 0.15 | 3.0 |
| Amitriptyline | 0.50 | 0.60 |
| Fluoxetine | 4.5 | 0.14 |
| Example 1 | 0.22 | 0.17 |
| Example 2 | 3.14 | 0.53 |
| Example 3 | 2.25 | 0.44 |
| Example 4 | 0.92 | 0.18 |
| Example 5 | 0.81 | 0.17 |
| Example 6 | 3.14 | 0.64 |
| Example 7 | 2.35 | 1.2 |
| Example 8 | 2.5 | 0.34 |
| Example 9 | 3.78 | 0.49 |
| Example 10 | 1.16 | 0.7 |
| Example 11 | 1.16 | 0.9 |
| Example 12 | 1.58 | 0.97 |

Inhibition of $^3$H-QNB binding: In the QNB receptor binding assay, the Compounds from Examples 1 and 2 exhibited an $IC_{50}$ greater than $10^{-5}$ molar and were therefore essentially inactive. Imipramine and DMI exhibit $K_i$'s of 37nM and 50nM, respectively. These results suggest that, unlike the tricyclic antidepressants, Compounds of Example 1 and 2 would have no muscarinic anticholinergic actions.

Hence, the end compounds of this invention are useful in the treatment of depression, for which purpose they may be administered orally or parenterally in an amount sufficient to alleviate the symptoms of depression. The actual amount of antidepressant agent to be used will vary with the severity and nature of the depressed state, the animal being treated and the level of relief sought. In the human, an oral dose of from about 2 to about 50 milligrams, administered as needed represents appropriate posology. Intramuscular administration of from about 1 to about 25 milligrams provides a dosage comparable to that specified for oral administration. As with other antidepressants, therapy should be initiated with lower dosages and increased until the desired symptomatic relief is obtained.

Pharmaceutical compositions containing the antidepressant compounds of this invention represent an additional aspect of this invention. The active ingredient can be compounded into any of the usual oral dosage forms including tablets, capsules and liquid preparations such as elixirs and suspensions containing various colouring, flavouring, stabilising and flavour masking substances. For compounding oral dosage forms, the active ingredient can be mixed with various conventional tabletting materials such as starch, calcium carbonate, lactose, sucrose and dicalcium phosphate to aid the tabletting or capsulating process. Magnesium stearate, as an additive, provides a useful lubricant function when desired.

The active ingredients can be dissolved or suspended in a pharmaceutically acceptable sterile liquid carrier, such as sterile water, sterile organic solvent or a mixture of both. Preferably a liquid carrier is one

suitable for parenteral injection. Where the active ingredient is sufficiently soluble it can be dissolved in normal saline as a carrier; if it is too insoluble for this it can often be dissolved in a suitable organic solvent, for instance aqueous propylene glycol or polyethylene glycol solutions. Aqueous propylene glycol containing from 10 to 75% of the glycol by weight is generally suitable. In other instances other compositions can be made by dispersing the finely-divided active ingredient in aqueous starch or sodium carboxymethylcellulose solution, or in a suitable oil, for instance arachis oil. Liquid pharmaceutical compositions which are sterile solutions or suspensions can be utilised by intramuscular, intraperitoneal or subcutaneous injection.

Accordingly this invention provides a pharmaceutical composition comprising a compound of formula I or a pharmaceutically acceptable salt thereof and a pharmaceutically acceptable carrier.

Preferably the pharmaceutical composition is in unit dosage form, e.g. as tablets or capsules. In such form, the composition is sub-divided in unit doses containing appropriate quantities of the active ingredient; the unit dosage forms can be packaged compositions, for example, packeted compositions, for example, packeted powders or ampoules. The unit dosage form can be a capsule, cachet or tablet itself, or it can be the appropriate number of any of these in package form. The quantity of the active ingredient in a unit dose of composition may be varied or adjusted from 2 mg. or less to 50 mg. or more, according to the particular need and the activity of the active ingredient.

The following examples illustrate the preparative technique employed in production of the compounds of the invention.

## Example 1

### 1-[1-(4-Chlorophenoxy)-2-(dimethylamino)ethyl]cyclohexanol

Para chlorophenoxy acetic acid (25 g, 0.134 mole) was dissolved in methylene chloride (500 ml) and treated with oxalyl chloride (13.4 ml, 0.15 mole) and dimethylformamide (0.5 ml) at room temperature. The mixture was stirred for three hours until gas evolution ceased. The solvent was evaporated and the residue dried under vacuum to remove excess oxalyl chloride. The residue was dissolved in methylene chloride (300 ml) and treated with gaseous dimethylamine. The mixture was stirred overnight and the solvent evaporated. The residue was redissolved in methylene chloride (200 ml) and the solution washed with saturated sodium bicarbonate solution, water,N-hydrochloric acid, water, brine, dried over magnesium sulfate and evaporated. The product, 2-(4-chlorophenoxy)-N,N-dimethyl acetamide, crystallized and was washed with hexane and air dried. Yield: 17.2 g, m.p. 69-71 °C.

| | |
|---|---|
| Analysis for: | $C_{10}H_{12}NO_2Cl$ |
| Calculated: | C, 56.21;H,5.66;N,6.56 |
| Found: | C, 55.65;H,5.63;N,6.57. |

Lithium di-isopropylamide was prepared by dissolving di-isopropylamine (11 ml) in tetrahydrofuran (150 ml) followed by the addition of n-butyl lithium (50 ml of 1.7 molar). After 10 minutes stirring, the straw coloured liquid was cooled to -78 °C and a solution of 2-(4-chlorophenoxy)-N,N-dimethylacetamide (16g, 0.074 mole) in tetrahydrofuran (25 ml) was slowly added. The mixture was stirred for 20 minutes at -78 °C and cyclohexanone (7 ml) added. After 45 minutes at -78 °C, the reaction mixture was poured into saturated ammonium chloride solution and a red colour ensued. The phases were separated and the organic layer washed with brine, dried over anhydrous potassium carbonate and evaporated giving 1-[(4-chlorophenoxy)(-(dimethyl-amino)carbonyl)-methyl]cyclohexanol as a red solid. Repeated washing with a cold hexane-isopropanol mixture yielded 2.7g of a white crystalline solid. Yield: 2.7 g, m.p. 109-111 °C.

| | |
|---|---|
| Analysis for: | $C_{16}H_{22}NO_3Cl$ |
| Calculated: | C,61.63; H,7.11; N,4.49 |
| Found: | C,61.01; H,6.91; N,4.63. |

Lithium aluminium hydride (0.6g) was suspended in dry tetrahydrofuran (20 ml) cooled to 0 °C and concentrated sulfuric acid (0.42 ml) was cautiously added in an *in situ* preparation of aluminium hydride. The mixture was stirred for one hour at 0 °C and 1-[(4-chlorophenoxy)((dimethylamino)carbonyl)methyl]-

cyclohexanol (2.7g, 0.009 mole) was dissolved in tetrahydrofuran (10 ml) and added. The reaction mixture was maintained below 10°C and stirred for one hour. The reaction mixture was cooled to 0°C and a tetrahydrofuran-water mixture (5 ml, 1:1 v/v) added slowly. Ten per cent sodium hydroxide (5 ml) was next added and the mixture filtered. The filtrate was dried over anhydrous potassium carbonate and evaporated. The residue was dissolved in ethyl acetate and the solution treated with 4N-isopropanolic HCl whereupon the title compound as the hydrochloride salt separated. The salt was filtered, washed with acetone, ethyl acetate, diethyl ether and petroleum ether and air dried. Yield: 530 mg, m.p. 218-220°C.

| | |
|---|---|
| Analysis for: | $C_{16}H_{24}NO_2Cl.HCl.\frac{1}{2}H_2O$ |
| Calculated: | C,55.98; H,7.63;N,4.08 |
| Found: | C,55.97; H,7.26;N,3.9. |

NMR Analysis (DMSO): 7.28 (4H quartet, aromatic) 4.72 (1H triplet, O-CH-$CH_2$-) 3.5 (2H doublet, O-CH-$CH_2$) 2.86 (6H singlet, -$N(CH_3)_2$) 1.4 (10H multiplet, aliphatic cyclohexyl)ppm.

Example 2

1-[1-(2,4-Dichlorophenoxy)-2-(dimethylamino)ethyl]cyclohexanol

By replacing p-chlorophenoxy acetic acid in Example 1 with a molar equivalent amount of 2,4-dichlorophenoxy acetic acid and following the procedure described therein, 1-[1-(2,4-dichlorophenoxy)-2-(dimethylamino)ethyl]cyclohexanol was obtained and converted to the hydrochloride salt using 4 N-isopropanolic HCl, m.p. 230-232°C.

| | |
|---|---|
| Analysis for: | $C_{16}H_{23}NO_2Cl_2.HCl$ |
| Calculated: | C,52.18;H,6.55;N,3.79 |
| Found: | C,51.97;H,6.39;N,3.78 |

Prepared as intermediate in this reaction was 1-[(2,4-dichlorophenoxy)((dimethylamino)carbonyl)methyl]-cyclohexanol.

Example 3

1-[1-(2,4-Dichlorophenoxy)-2-(dimethylamino)ethyl]cyclopentanol

By replacing cyclohexanone in Example 2 with a molar equivalent amount of cyclopentanone, the title compound was obtained and converted to the hydrochloride salt using 4N-isopropanolic HCl, m.p. 174-175°C.

| | |
|---|---|
| Analysis for: | $C_{15}H_{21}NO_2Cl_2.HCl$ |
| Calculated: | C,50.79; H,6.25; N,3.95 |
| Found: | C,50.81; H,6.24; N,3.97. |

Prepared as intermediate in this reaction was 1-[(2,4-dichlorphenoxy)(dimethylamino)carbonyl)methyl]-cyclopentanol.

Example 4

1-[2-(Dimethylamino)-1-(4-methoxyphenoxy)ethyl]cyclohexanol

By replacing p-chlorophenoxy acetic acid with a molar equivalent amount of p-methoxyphenoxy acetic acid in Example 1 and following the procedure described therein, 1-[[(dimethylamino)carbonyl][4-methoxyphenoxy]methyl]cyclohexanol was obtained as a crystalline solid, m.p. 98-99° C.

| | |
|---|---|
| Analysis for: | $C_{17}H_{25}NO_4$ |
| Calculated: | C,66.42;H,8.20;N,4.86 |
| Found: | C,66.14;H,8.12;N,4.54. |

To a solution of Borane/tetrahydrofuran complex (50 ml, 50 mmole) was added a solution of 1-[[-(dimethylamino)carbonyl][4-methoxyphenoxy]methyl]cyclohexanol (5g, 16.3 mmole) in dry tetrahydrofuran (25 ml). The mixture was refluxed for one hour, and cooled in an ice bath. 6N HCl (18 ml) was added and the solution refluxed for one hour. The solution was then cooled in an ice bath, basified with solid potassium hydroxide and the two layers separated. The organic layer was washed with brine, dried over magnesium sulfate and evaporated. The solid residue was dissolved in diethyl ether and 4N-isopropanolic HCl was added. The title compound, as the hydrochloride salt, was washed well with diethyl ether, acetone, ethyl acetate and petroleum ether, and dried in a desiccator under vacuum. Yield: 3.52g; m.p. 196-198° C.

| | |
|---|---|
| Analysis for: | $C_{17}H_{27}NO_3$.HCl |
| Calculated: | C,61.90;H,8.56;N,4.25 |
| Found: | C,61.52;H,8.51;N,4.36. |

NMR Analysis (DMSO): 7.0 (4H, aromatic) 4.38 (1H,OCH-$CH_2$) 3.76 (3H singlet, -OMe) 3.54 (2H,)-CH-$CH_2$) 2.86 (6H singlet, $N(CH_3)_2$) 1.5 (10H multiplet, aliphatic cyclohexyl) ppm.

Example 5

1-[2-(Dimethylamino)-1-(4-methoxyphenoxy)ethyl]cycloheptanol

By replacing cyclohexanone with a molar equivalent amount of cycloheptanone in Example 4, the title compound was obtained and converted to the hydrochloride, m.p. 185-186° C.

| | |
|---|---|
| Analysis for: | $C_{18}H_{29}NO_3$.HCl |
| Calculated: | C,62.86;H,8.79;N,4.07 |
| Found: | C,62.87;H,8.84;N,3.92. |

Prepared as intermediate in this reaction was 1-[[(dimethylamino)carbonyl][4-methoxyphenoxy]methyl]-cycloheptanol.

Example 6

1-[2-(dimethylamino)-1(4-methoxyphenoxy)ethyl]cyclopentanol

By replacing cyclohexanone with a molar equivalent amount of cyclopentanone in Example 4 the title compound was obtained and converted to the hydrochloirde, m.p. 167-168° C.

| | |
|---|---|
| Analysis for: | $C_{16}H_{25}NO_3$.HCl |
| Calculated: | C,60.85;H,8.30;N,4.43 |
| Found: | C,60.37;H,8.20;N,4.57. |

Prepared as intermediate in this reaction was 1-[[(dimethylamino)carbonyl][4-methoxyphenoxy]methyl]-

cyclopentanol.

Example 7

1-[2-(dimethylamino)-1-(3-trifluoromethylphenoxy)ethyl]cyclohexanol

m-Hydroxybenzotrifluoride (8.5ml, 70 mmole) was dissolved in absolute ethanol (50 ml) and solid potassium hydroxide (3.92 g) added. The mixture was stirred until solution was complete and the solvent was evaporated. The residue, a red oil, was dissolved in 2-butanone (80 ml). Potassium iodide (2g) and 2-chloro-N,N-dimethylacetamide (7.9g, 65 mmole) were added and the mixture refluxed overnight. The reaction mixture was then cooled in ice and filtered. The filtrate was evaporated and the residue obtained partitioned between diethyl ether and 5% sodium hydroxide. The organic layer was washed with water, brine, dried over magnesium sulfate and evaporated. N,N-dimethyl-2-[3-(trifluoromethyl)phenoxy]acetamide was obtained as a yellow solid. Yield: 13.8 g, m.p. 88.5-89.5 ° C.

| | |
|---|---|
| Analysis for: | $C_{11}H_{12}NO_2F_3$ |
| Calculated: | C,53.44;H,4.89;N,5.67 |
| Found: | C,53.01;H,4.85;N,5.55. |

N,N-dimethyl-2-[3-(trifluoromethyl)phenoxy]acetamide, prepared in the preceding paragraph was reacted with cyclohexanone following the procedure of Example 1 to give 1-[[(dimethylamino)carbonyl][3-trifluoromethylphenoxy] methyl]cyclohexanol which was reduced with borane/tetrahydrofuran as in Example 3. The title compound was obtained and its hydrochloride salt prepared,m.p.210-211 ° C.

| | |
|---|---|
| Analysis for: | $C_{17}H_{24}NO_2F_3$.HCl |
| Calculated: | C,55.51;H,6.85;N,3.81 |
| Found: | C,55.75;H,6.81;N,4.04. |

Example 8

1-[2-Dimethylamino-1-(4-trifluoromethylphenoxy)ethyl]cyclohexanol

By replacing m-hydroxybenzotrifluoride with a molar equivalent amount of p-hydroxybenzotrifluoride in Example 7, the title compound was obtained and its hydrochloride salt prepared, m.p. 251-252 ° C.

| | |
|---|---|
| Analysis for: | $C_{17}H_{24}NO_2F_3$.HCl |
| Calculated: | C,55.56;H,6.80;N,3.87 |
| Found: | C,55.36;H,6.78;N,3.72. |

Prepared as intermediate in this reaction was 1-[[(dimethylamino)carbonyl][4-trifluoromethylphenoxy)-methyl]cyclohexanol.

Example 9

1-[2-Dimethylamino-1-(4-trifluoromethylphenoxy)ethyl]cycloheptanol

By replacing cyclohexanone with a molar equivalent amount of cycloheptanone in Example 7, the title compound was obtained and characterized as its hydrochloride salt, m.p. 210-212 ° C.

Analysis for: $C_{18}H_{26}NO_2F_3.HCl.\frac{1}{2}C_3H_8O$
Calculated: C,56.86; H,7.59;N,3.40
Found: C,57.34;H,7.40;N,3.47.

Prepared as intermediate in this reaction was 1-[[(dimethylamino)carbonyl][4-trifluoromethylphenoxy)-methyl]cycloheptanol.

Example 10

1-[2-(Dimethylamino)-1-(3-methoxyphenoxy)ethyl]cyclohexanol

By replacing p-methoxyphenoxy acetic acid with a molar equivalent of m-methoxyphenoxy acetic acid in Example 4, the title compound was obtained and the hydrochloride prepared.

Analysis for: $C_{17}H_{27}NO_3.HCl$
Calculated: C,61.90;H,8.56;N,4.25
Found: C,61.45;H,8.46;N,4.51.

Prepared as intermediate in this reaction was 1-[[(dimethylamino)carbonyl][3-methoxyphenoxy]methyl]-cyclohexanol.

Example 11

1-[2-Dimethylamino)-1-(3-methoxyphenoxy)ethyl]cycloheptanol

By replacing cyclohexanone with a molar equivalent amount of cycloheptanone in Example 10, the title compound was obtained and its hydrochloride salt prepared.

Analysis for: $C_{18}H_{29}NO_3.HCl$
Calculated: C,62.87;H,8.5;N,4.07
Found: C,62.16;H,8.6;N,4.13.

Prepared as intermediate in this reaction was 1-[2-dimethylamino)-1-(3-methoxyphenoxy)ethyl]-cycloheptanol.

Example 12

1-[2-(Dimethylamino)-1-(3-methoxyphenoxy)ethyl]cyclopentanol

By replacing cyclohexanone with a molar equivalent of cyclopentanone in Example 10, the title compound was obtained and its hydrochloride prepared, m.p. 168-169°C.

Analysis for: $C_{16}H_{25}NO_3.HCl$
Calculated: C,60.85; H,8.30; N,4.43
Found: C,60.38; H,8.28; N,4.62.

Prepared as intermediate in this reaction was 1-[2-dimethylamino)-1-(3-methoxyphenoxy)ethyl]-cyclopentanol.

Example 13

1-[2-dimethylamino-1-(4-methoxyphenoxy)ethyl]cyclohexanol

1-oxaspiro[2,5]octane-2-carbonitrile

Cyclohexanone (11.4ml. 0.11 mole) was combined with 50% aqueous NaOH (20 ml) and triethylamine (TEA) (0.4g) and vigorously stirred for 10 minutes. The reaction was cooled to 15° C and treated dropwise with chloroacetonitrile (6.33ml, 0.1 mole). The reaction was flooded with water, and the product was extracted with ethyl acetate. The organic layer was then washed with 1N HCl, $H_2O$, saturated $NaHCO_3$, $H_2O$, and brine, dried over $MgSO_4$ and decolourised with carbon. The mixture was filtered, and the solvent was removed yielding a brown oil. This oil may be purified by distillation. (b.p. 87° /5 mm Hg) 9.8g (71.5% of theory) of 1-oxaspiro[2,5]octane-2-carbonitrile were realized.
I.R. - 2250, 1705, 915, 805 $cm^{-1}$
N.M.R. - 1.71 (d), 2.36(p).

1-[1-cyano-(4-methoxyphenoxy)methyl]cyclohexanol

Method A

4-Methoxyphenol (4.7g, 0.038 mole) was combined with KOH (2.15g, 0.039 mole) in 95% ethanol (50 ml). After stirring for 30 minutes, the ethanol was removed by rotary evaporation yielding an oily phenate. The phenate was redissolved in tetrahydrofuran (THF) (80 ml) and treated with 1-oxaspiro[2,5]octane-2-carbonitrile (4.8g, 0.035 mole) which had been dissolved in 20 ml of THF. The solution was refluxed overnight and then iced. The THF was removed by rotary evaporation, and the oily residue was partitioned between ethyl acetate and water. The organic layer was then washed with 1N HCl, $H_2O$, saturated $NaHCO_3$, $H_2O$, and brine, dried over $MgSO_4$ and decolourised with carbon. The mixture was filtered, and the solvent was removed yielding a brown oil 4.2g.

Method B

4-Methoxyphenol (4.79, 0.038 mole) was dissolved in ethanol (50 ml) and treated with NaOH (0.5g). The mixture was refluxed for 15 minutes and then cooled. 1-oxaspiro[2,5]octane-2-carbonitrile (4.8g, 0.035 mole) were dissolved in ethanol (50 ml) and added to the phenate solution. The flask was filled with $N_2$, stoppered and allowed to stand at 5° C overnight. The reaction mixture was then heated at 55° C for 3 hours, and then refluxed at 78° C for 2½ hours. The reaction was then iced and the ethanol evaporated. The oily residue was partitioned between ethyl acetate and water. The organic layer was then washed with 1N HCl, $H_2O$, saturated $NaHCO_3$, $H_2O$, and brine, dried over $MgSO_4$ and decolourised with carbon. The mixture was filtered, and the solvent was remvoed yielding a brown oil, 5.5g (60.11% of theory).
Mass Spec - 262 (M+1)

| Elemental Analysis - | | | |
|---|---|---|---|
| Calculated: | C,68.94; | H,7.33; | N,5.36 |
| Found: | C,68.79; | H,7.19; | N,5.14 |

1-[2-amino-1-(4-methoxyphenoxy)ethyl]cyclohexanol

1-[1-Cyano-(4-methoxyphenoxy)methyl]cyclohexanol (4.29. 0.016 mole) was dissolved in dry THF (100 ml) and treated with borane/THF complex (35 ml, 0.035 mole). The solution was refluxed for 4 hours and cooled in ice. The reaction mixture was then treated with 2N NaOH (70 ml) and stirred for 30 minutes. The layers were separated, and the organic layer was washed in brine and dried over $MgSO_4$. The solvent was evaporated giving 4.1g of the title product as an oil.
Mass Spec. - 266 (M+1)

| | |
|---|---|
| Analysis for: | $C_{15}H_{23}NO_3$ |
| Calculated: | C,67.9; H, 8.74; N, 5.2 |
| Found: | C,65.55; H, 8.73; N, 3.87. |

1-[2-dimethylamino-1-(4-methoxyphenoxy)ethyl]cyclohexanol

The product of the preceding paragraph was dissolved in a mixture of formaldehyde, formic acid and water and the solution was refluxed overnight (Eschweiler-Clark reaction). The reaction mixture was cooled, basified with KOH and extracted with methylene chloride. The extract was washed with brine, dried over magnesium sulphate and taken to dryness. The product was taken up in diethyl ether and converted to the hydrochloride salt with 3N-isopropanolic HCl to yield the title compound which was confirmed to be identical to that obtained in Example 4.

## Claims

**Claims for the following Contracting States : BE, CH, DE, FR, IT, LI, LU, NL, SE**

**1.** A compound of formula I

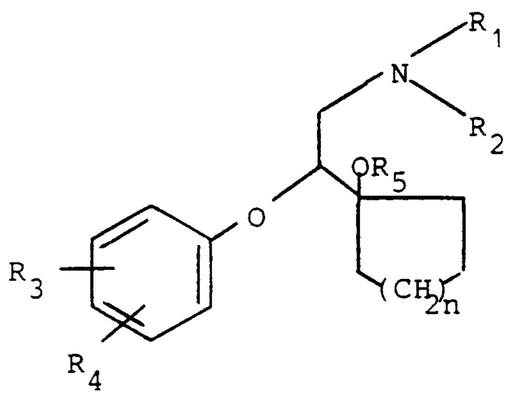

(I)

in which

$R_1$ is hydrogen or alkyl of 1 to 6 carbon atoms;

$R_2$ is alkyl of 1 to 6 carbon atoms;

$R_3$ and $R_4$ are independently hydrogen, hydroxyl, alkyl of 1 to 6 carbon atoms, alkoxy of 1 to 6 carbon atoms, alkanoyloxy of 2 to 7 carbon atoms, halo or trifluoromethyl;

$R_5$ is hydrogen, alkyl of 1 to 6 carbon atoms or alkanoyl of 2 to 7 carbon atoms;

and n is one of the integers 0,1,2 or 3;

or a pharmaceutically acceptable salt thereof.

**2.** A compound of formula II

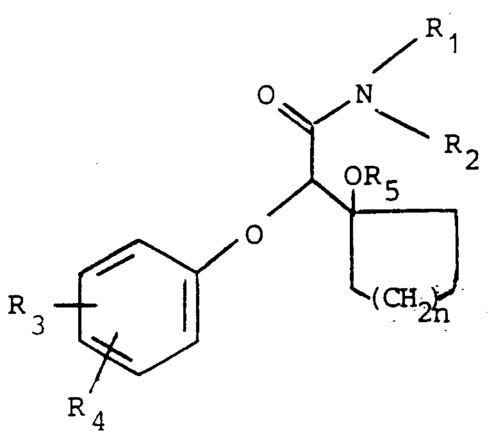

(II)

wherein n, $R_1$, $R_2$, $R_3$ and $R_4$ are as defined in Claim 1 and $R_5$ is hydrogen or alkyl of 1 to 6 carbon atoms.

3. A compound of formula I or II according to Claim 1 or Claim 2 wherein $R_1$ and/or $R_2$ are/is alkyl of 1 to 3 carbon atoms.

4. A compound of formula I or II according to any one of Claims 1 to 3 wherein $R_3$ is hydrogen, alkoxy of 1 to 3 carbon atoms, chloro, bromo or trifluoromethyl.

5. A compound of formula I or II according to any one of Claims 1 to 4 wherein $R_4$ is alkyl of 1 to 3 carbon atoms, alkoxy of 1 to 3 carbon atoms, chloro, bromo or trifluoromethyl.

6. A compound of formula I or II according to any one of Claims 1 to 5 wherein $R_3$ and $R_4$ are in meta or para positions.

7. A compound as claimed in Claim 1 which is 1-[1-(4-chlorophenoxy)-2-(dimethylamino)ethyl]-cyclohexanol;

1-[1-(2,4-dichlorophenoxy)-2-(dimethylamino)ethyl]cyclohexanol;

1-[1-(2,4-dichlorophenoxy)-2-(dimethylamino)ethyl]cyclopentanol;

1-[2-(dimethylamino)-1-(4-methoxyphenoxy)ethyl]cyclohexanol;

1-[2-(dimethylamino)-1-(4-methoxyphenoxy)ethyl]cycloheptanol;

1-[2-(dimethylamino)-1-(4-methoxyphenoxy)ethyl]cyclopentanol;

1-[2-(dimethylamino)-1-(3-trifluoromethylphenoxy)ethyl]cyclohexanol;

1-[2-(dimethylamino)-1-(4-trifluoromethylphenoxy)ethyl]cyclohexanol;

1-[2-(dimethylamino)-1-(4-trifluoromethylphenoxy)ethyl]cycloheptanol;

1-[2-(dimethylamino)-1-(3-methoxyphenoxy)ethyl]cyclohexanol;

1-[2-(dimethylamino)-1-(3-methoxyphenoxy)ethyl]cycloheptanol or

1-[2-(dimethylamino)-1-(3-methoxyphenoxy)ethyl]cyclopentanol or a pharmaceutically acceptable salt thereof.

8. A compound as claimed in Claim 2 which is 1-[(4-chlorophenoxy)((dimethylamino)carbonyl)methyl]-cyclohexanol;

1-[(2,4-dichlorophenoxy)](dimethylamino)carbonyl)methyl]cyclohexanol;

1-[(2,4-dichlorophenoxy)((dimethylamino)carbonyl)methyl]cyclopentanol;

1-[[(dimethylamino)carbonyl][4-methoxyphenoxy]methyl]cyclohexanol;

1-[[(dimethylamino)carbonyl][4-methoxyphenoxy]methyl]cycloheptanol;

1-[[(dimethylamino)carbonyl] [4-methoxyphenoxy]methyl]cyclopentanol;

1-[[dimethylamino)carbonyl][3-trifluoromethylphenoxy]methyl]cyclohexanol;

1-[[dimethylamino)carbonyl][4-trifluoromethylphenoxy]-methyl]cyclohexanol;

1-[[dimethylamino)carbonyl][4-trifluoromethylphenoxy]-methyl]cycloheptanol;

1-[[(dimethylamino)carbonyl][3-methoxyphenoxy]methyl]cyclohexanol;

1-[[dimethylamino)carbonyl][3-methoxyphenoxy]methyl]cycloheptanol or

1-[[(dimethylamino)carbonyl][3-methoxyphenoxy]methyl]cyclopentanol.

**9.** A process for preparing a compound of formula I or a pharmaceutically acceptable salt thereof as defined in Claim 1 which comprises:

(i) reducing a compound of formula A

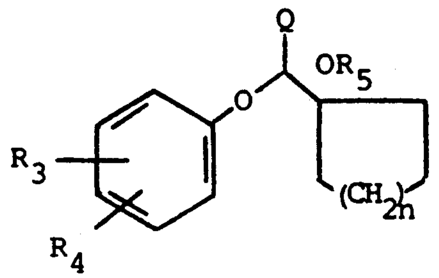

(A)

where Q represents $-CONR_1R_2$; $-CH=NR_2$; $-CH(OH)NR_1R_2$; $-CH_2NR_1COR_9$; $-CH_2N=CY_1Y_2$ - $CH_2NR_1CH(OH)R_8$,

$R_9$ represents alkoxy, $C_{1-5}$ alkyl or hydrogen;

$Y_1$ and $Y_2$ are each selected from hydrogen and alkyl such that the group $=CY_1Y_2$ contains 1 to 6 carbon atoms

$R_8$ is $C_1$-$C_5$ alkyl

and n,$R_1$,$R_2$,$R_3$,$R_4$ and $R_5$ are as defined in Claim 1 or

(2) (a) reacting a compound having formula B

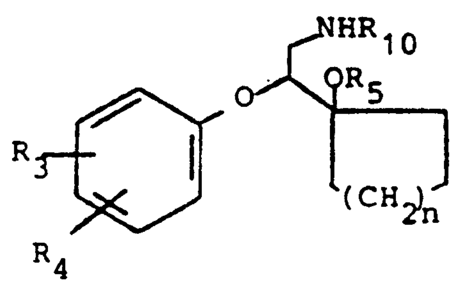

(B)

(where $R_{10}$ is $R_1$ or a readily splittable substituent, and $R_3$, $R_4$ and $R_5$ are as defined in Claim 1 with an alkylating agent to introduce one or two $C_1$-$C_6$ alkyl groups (if $R_{10}$ is H) or one such alkyl group and, if necessary, splitting off the said readily splittable substituent;

or (b) reacting a compound of formula B as defined above wherein $R_{10}$ is $R_1$ with a carbonyl compound of formula $Y_1Y_2CO$ [where $Y_1$ and $Y_2$ are as defined above] in the presence of a reducing agent; or

(3) (a) reacting a compound of formula $HNR_2R_{10}$ (where $R_2$ and $R_{10}$ are as defined above) with a compound having the formula C

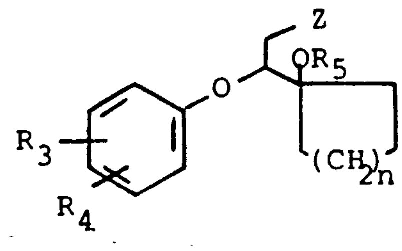

(C)

(where Z is a leaving group and $R_3$, $R_4$ and $R_5$ are as defined under formula I and, if necessary, splitting off the substituent $R_{10}$;
or (b) reacting a compound of formula $HNR_1R_2$ (where $R_1$ and $R_2$ are as defined above) with an aldehyde of formula D

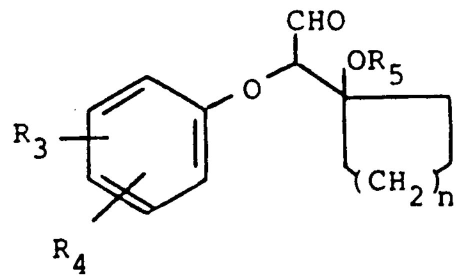

(D)

(where $R_3$, $R_4$, $R_5$ and n are as defined in Claim 1 in the presence of a reducing agent or
or
(4) reacting a compound of formula I wherein $R_5$ is hydrogen with a reactive derivative of a $C_{2-7}$ alkanoic acid to introduce $C_{2-7}$ alkanoyl as $R_5$;
or
(5) forming a compound of formula I or salt thereof by removal of a protecting group to give hydroxy as $R_3$ and/or $R_4$ and/or $OR_5$
or
(6) reacting a compound of formula I in free base form with an acid to form a pharmaceutically acceptable salt thereof or reacting a salt of a compound of formula I with a base to give the compound of formula I in free base form.

**10.** A process for the preparation of a compound of formula II as defined in Claim 2 which comprises
(a) reacting an anion of formula E

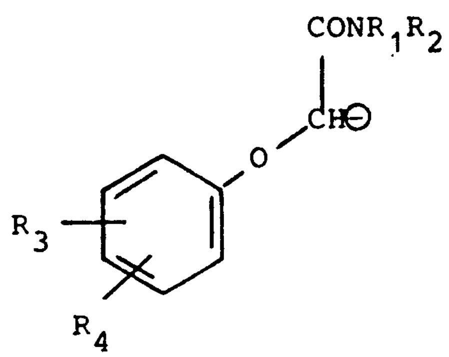

(E)

(where $R_1$, $R_2$, $R_3$ and $R_4$ are as defined in Claim 1 with a cycloalkanone the formula F

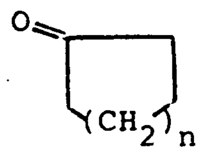

(F)

(where n is as defined in Claim 1 and, if desired, introducing an alkyl $R_5$ group by alkylation; or
(b) reacting an amine having the formula $HNR_1R_2$ (where $R_1$ and $R_2$ are as defined above) with the acid halide derivative, active ester or anhydride of an acid having the formula (G)

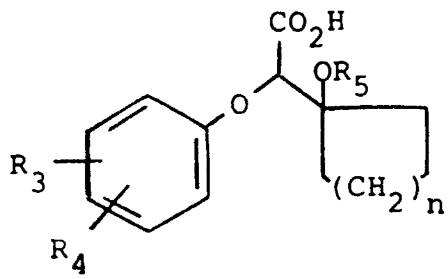

(G)

(where $R_3$, $R_4$, $R_5$ and n are as defined in Claim 2.

**11.** A compound of formula I as claimed in any one of Claims 1, and 3 to 7 for use as a pharmaceutical.

**12.** A pharmaceutical composition comprising a compound of formula I or a pharmaceutically acceptable salt thereof according to any one of Claims 1 and 3 to 7 and a pharmaceutically acceptable carrier.

**Claims for the following Contracting State: AT**

**1.** A process for preparing a compound of formula I

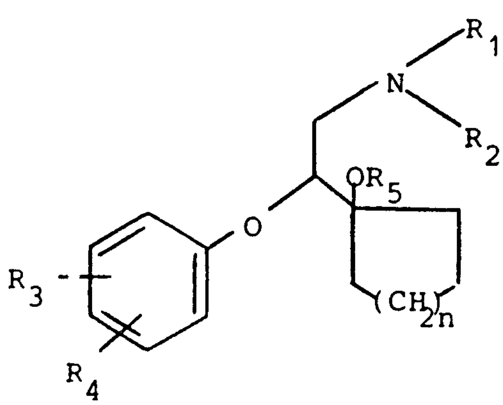

(I)

in which
$R_1$ is hydrogen or alkyl of 1 to 6 carbon atoms;
$R_2$ is alkyl of 1 to 6 carbon atoms;
$R_3$ and $R_4$ are independently hydrogen, hydroxyl, alkyl of 1 to 6 carbon atoms, alkoxy of 1 to 6 carbon atoms, alkanoyloxy of 2 to 7 carbon atoms, halo or trifluoromethyl;
$R_5$ is hydrogen, alkyl of 1 to 6 carbon atoms or alkanoyl of 2 to 7 carbon atoms;
and n is one of the integers 0,1,2 or 3;
or a pharmaceutically acceptable salt thereof,
which comprises
(i) reducing a compound of formula A

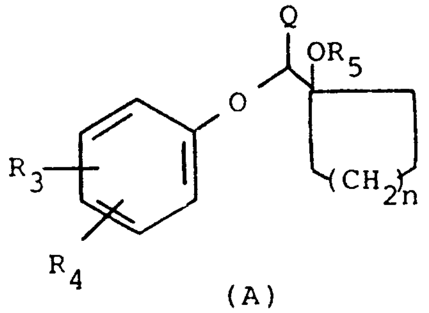

(A)

where Q represents $-CONR_1R_2$; $-CH=NR_2$; $-CH(OH)NR_1R_2$; $-CH_2NR_1COR_9$; $-CH_2N=CY_1Y_2$ - $CH_2NR_1CH(OH)R_8$,

$R_9$ represents alkoxy, $C_{1-5}$ alkyl or hydrogen;

$Y_1$ and $Y_2$ are each selected from hydrogen and alkyl such that the group $=CY_1Y_2$ contains 1 to 6 carbon atoms,

$R_8$ is $C_1$-$C_5$ alkyl

and n, $R_3$, $R_4$ and $R_5$ are as defined above;

or

(ii)(a) reacting a compound having formula B

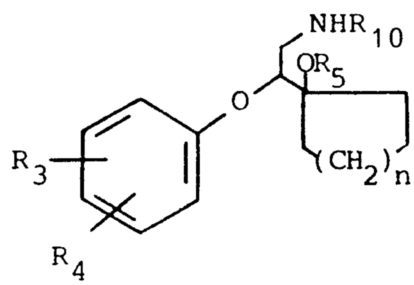

(where $R_{10}$ is $R_1$ or a readily splittable substituent, and $R_3$, $R_4$ and $R_5$ are as defined above with an alkylating agent to introduce one or two $C_1$-$C_6$ alkyl groups (if $R_{10}$ is H) or one such alkyl group and, if necessary, splitting off the said readily splittable substituent;

or (b) reacting a compound of formula B as defined above wherein $R_{10}$ is $R_1$ with a carbonyl compound of formula $Y_1Y_2CO$ [where $Y_1$ and $Y_2$ are as defined above] in the presence of a reducing agent; or

(iii)(a) reacting a compound of formula $HNR_2R_{10}$ (where $R_2$ and $R_{10}$ are as defined above) with a compound having the formula C

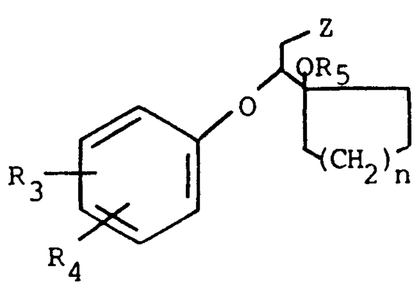

(C)

(where Z is a leaving group and $R_3$, $R_4$ and $R_5$ are as defined under formula I and, if necessary, splitting off the substituent $R_{10}$;

or (b) reacting a compound of formula $HNR_1R_2$ (where $R_1$ and $R_2$ are as defined above) with an aldehyde of formula D

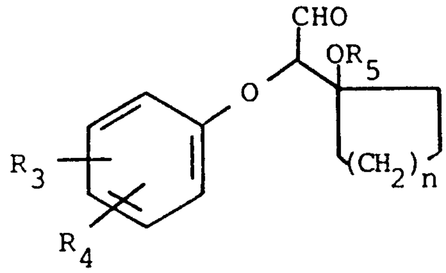

(D)

(where $R_3$, $R_4$, $R_5$ and n are as defined above in the presence of a reducing agent;
or
(iv) reacting a compound of formula I wherein $R_5$ is hydrogen with a reactive derivative of a $C_{2-7}$ alkanoic acid to introduce $C_{2-7}$ alkanoyl as $R_5$;
or
(v) forming a compound of formula I or salt thereof by removal of a protecting group to give hydroxy as $R_3$ and/or $R_4$ and/or $OR_5$;
or
(vi) reacting a compound of formula I in free base form with an acid to form a pharmaceutically acceptable salt thereof or reacting a salt of a compound of formula I with a base to give the compound of formula I in free base form.

**2.** A process for the preparation of a compound of formula II

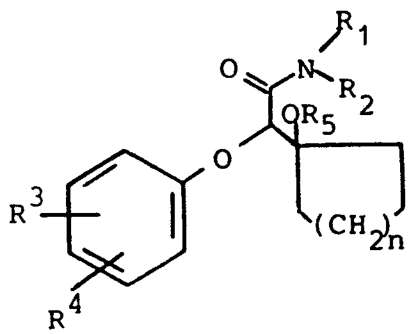

(II)

wherein n, $R_1$, $R_2$, $R_3$ and $R_4$ are as defined in Claim 1 in connection with formula I and $R_5$ is hydrogen or alkyl of 1 to 6 carbon atoms which comprises
(a) reacting an anion of formula E

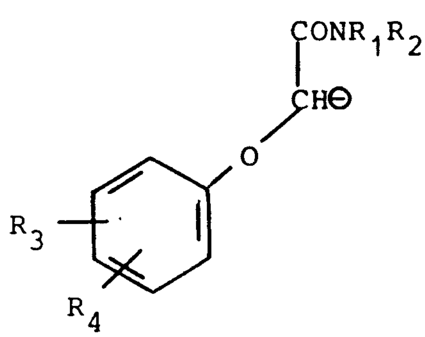

(E)

where $R_1$, $R_2$, $R_3$ and $R_4$ are as defined in Claim 1 with a cycloalkanone of formula F

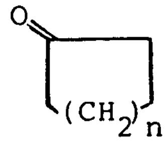

(F)

(where n is as defined in Claim 1 and, if desired, introducing an alkyl $R_5$ group by alkylation; or
(b) reacting an amine having the formula $HNR_1R_2$ (where $R_1$ and $R_2$ are as defined above) with the acid halide derivative, active ester or anhydride of an acid having the formula (G)

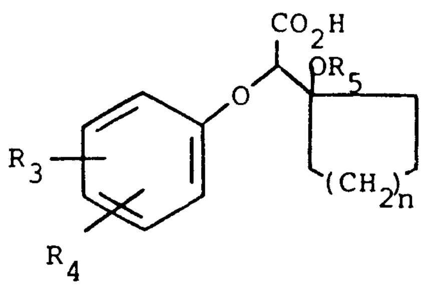

(G)

wherein n, $R_3$, $R_4$, and $R_5$ are as defined above.

**3.** A process as claimed in Claim 1 or Claim 2 wherein $R_1$ and/or $R_2$ are/is alkyl of 1 to 3 carbon atoms.

**4.** A process as claimed in any one of Claims 1 to 3 wherein $R_3$ is hydrogen, alkoxy of 1 to 3 carbon atoms, chloro, bromo or trifluoromethyl.

**5.** A process according to any one of Claims 1 to 4 wherein $R_4$ is alkyl of 1 to 3 carbon atoms, alkoxy of 1 t 3 carbon atoms, chloro, bromo or trifluoromethyl.

**6.** A process according to any one of Claims 1 to 5 wherein $R_3$ and $R_4$ are in meta or para positions.

**7.** A process as claimed in Claim 1 in which the compound prepared is 1-[1-(4-chlorophenoxy)-2-(dimethylamino)ethyl]-cyclohexanol;

1-[1-(2,4-dichlorophenoxy)-2-(dimethylamino)ethyl]cyclohexanol;

1-[1-(2,4-dichlorophenoxy)-2-(dimethylamino)ethyl]cyclopentanol;

1-[2-(dimethylamino)-1-(4-methoxyphenoxy)ethyl]cyclohexanol;

1-[2-(dimethylamino)-1-(4-methoxyphenoxy)ethyl]cycloheptanol;

1-[2-(dimethylamino)-1-(4-methoxyphenoxy)ethyl]cyclopentanol;

1-[2-(dimethylamino)-1-(3-trifluoromethylphenoxy)ethyl]cyclohexanol;

1-[2-(dimethylamino)-1-(4-trifluoromethylphenoxy)ethyl]cyclohexanol;

1-[2-(dimethylamino)-1-(4-trifluoromethylphenoxy)ethyl]cycloheptanol;

1-[2-(dimethylamino)-1-(3-methoxyphenoxy)ethyl]cyclohexanol;

1-[2-(dimethylamino)-1-(3-methoxyphenoxy)ethyl]cycloheptanol; or

1-[2-(dimethylamino)-1-(3-methoxyphenoxy)ethyl]cyclopentanol; or a pharmaceutically acceptable salt

thereof.

**8.** A process as claimed in Claim 2 in which the compound prepared is 1-[(4-chlorophenoxy)-(dimethylamino)carbonyl)methyl]-cyclohexanol;

1-[(2,4-dichlorophenoxy)(dimethylamino)carbonyl)methyl]cyclohexanol;

1-[(2,4-dichlorophenoxy)((dimethylamino)carbonyl)methyl]cyclopentanol;

1-[[(dimethylamino)carbonyl][4-methoxyphenoxy]methyl]-cyclohexanol;

1-[[(dimethylamino)carbonyl][4-methoxyphenoxy]methyl]-cycloheptanol;

1-[[dimethylamino)carbonyl][4-methoxyphenoxy]methyl]cyclopentanol;

1-[[dimethylamino)carbonyl][3-trifluoromethylphenoxy]-methyl]cyclohexanol; or

1-[[dimethylamino)carbonyl][4-trifluoromethylphenoxy]-methyl]cyclohexanol;

1-[[dimethylamino)carbonyl][4-trifluoromethylphenoxy]methyl]cycloheptanol;

1-[[dimethylamino)carbonyl][3-methoxyphenoxy]methyl]cyclohexanol;

1-[[(dimethylamino)carbonyl][3-methoxyphenoxy]methyl]cycloheptanol;or

1-[[(dimethylamino)carbonyl][3-methoxyphenoxy]methyl]cyclopentanol.

**9.** A process for preparing a pharmaceutical composition which comprises bringing a compound of formula I or a pharmaceutically acceptable salt thereof as defined in Claim 1 into association with a pharmaceutically acceptable carrier.

**10.** A process for preparing a pharmaceutical composition which comprises bringing a compound of formula I prepared according to any one of Claims 1, and 3 to 7 into association with a pharmaceutically acceptable carrier.

**Revendications**
**Revendications pour les Etats contractants suivants : BE, CH, DE, FR, IT, LI, LU, NL, SE**

**1.** Composé de formule I dans laquelle

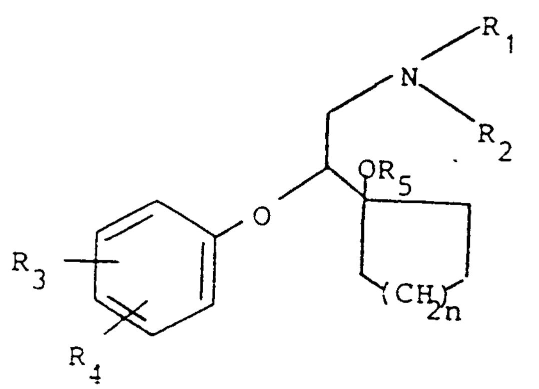

(I)

$R_1$ est l'hydrogène ou un groupe alkyle ayant 1 à 6 atomes de carbone ;
$R_2$ est un groupe alkyle ayant 1 à 6 atomes de carbone ;
$R_3$ et $R_4$ représentent indépendamment l'hydrogène, un groupe hydroxyle, alkyle ayant 1 à 6 atomes de carbone, alkoxy ayant 1 à 6 atomes de carbone, alcanoyloxy ayant 2 à 7

atomes de carbone, halogéno ou trifluorométhyle ;

$R_5$ est l'hydrogène, un groupe alkyle ayant 1 à 6 atomes de carbone ou alcanoyle ayant 2 à 7 atomes de carbone ;

et

n est l'un des nombres entiers 0, 1, 2 et 3 ;

ou un sel pharmaceutiquement acceptable de ce composé.

**2.** Compose de formule II

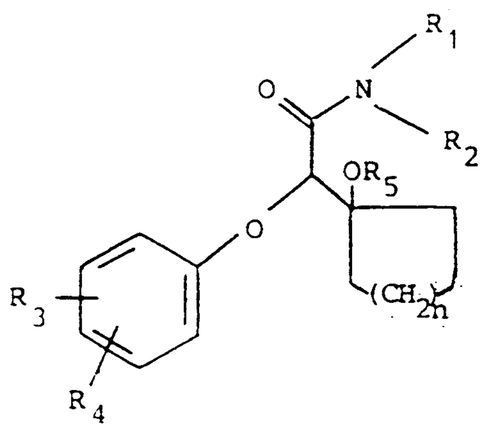

(II)

dans laquelle n, $R_1$, $R_2$, $R_3$ et $R_4$ sont tels que définis dans la revendication 1 et $R_5$ est l'hydrogène ou un groupe alkyle ayant 1 à 6 atomes de carbone.

**3.** Composé de formule I ou II suivant la revendication 1 ou la revendication 2, dans lequel $R_1$ et/ou $R_2$ représentent un groupe alkyle ayant 1 à 3 atomes de carbone.

**4.** Composé de formule I ou II suivant l'une quelconque des revendications 1 à 3, dans lequel $R_3$ est l'hydrogène, un groupe alkoxy de 1 à 3 atomes de carbone, chloro, bromo ou trifluorométhyle.

**5.** Composé de formule I ou II suivant l'une quelconque des revendications 1 à 4, dans lequel $R_4$ est un groupe alkyle ayant 1 à 3 atomes de carbone, alkoxy ayant 1 à 3 atomes de carbone, chloro, bromo ou trifluorométhyle.

**6.** Composé de formule I ou II suivant l'une quelconque des revendications 1 à 5, dans lequel $R_3$ et $R_4$ sont en positions méta ou para.

**7.** Composé suivant la revendication 1, qui est le 1-[1-(4-chlorophénoxy)-2-(diméthylamino)éthyl]-cyclohexanol ;
le 1-[1-(2,4-dichlorophénoxy)-2-(diméthylamino)éthyl]cyclohexanol ;
le 1-[1-(2,4-dichlorophénoxy)-2-(diméthylamino)éthyl]cyclopentanol ;
le 1-[2-(diméthylamino)-1-(4-méthoxyphénoxy)éthyl]cyclohexanol ;
le 1-[2-(diméthylamino)-1-(4-méthoxyphénoxy)éthyl]cycloheptanol ;
le 1-[2-(diméthylamino)-1-(4-méthoxyphénoxy)éthyl]cyclopentanol ;
le 1-[2-(diméthylamino)-1-(3-trifluorométhylphénoxy)éthyl]cyclohexanol ;
le 1-[2-(diméthylamino)-1-(4-trifluorométhylphénoxy)éthyl]cyclohexanol ;
le 1-[2-(diméthylamino)-1-(4-trifluorométhylphénoxy)éthyl]cycloheptanol ;
le 1-[2-(diméthylamino)-1-(3-méthoxyphénoxy)éthyl]cyclohexanol ;
le 1-[2-(diméthylamino)-1-(3-méthoxyphénoxy)éthyl]cycloheptanol ou
le 1-[2-(diméthylamino)-1-(3-méthoxyphénoxy)éthyl]cyclopentanol ou un sel pharmaceutiquement acceptable de ce composé.

**8.** Composé suivant la revendication 2, qui est le 1-[(4-chlorophénoxy)((diméthylamino)carbonyl)méthyl]-cyclohexanol ;
le 1-[(2,4-dichlorophénoxy)((diméthylamino)carbonyl)méthyl]cyclohexanol ;

le 1-[(2,4-dichlorophénoxy)((diméthylamino)carbonyl)méthyl]cyclopentanol ;
le 1-[[(diméthylamino)carbonyl][4-méthoxyphénoxy]méthyl]cyclohexanol ;
le 1-[[(diméthylamino)carbonyl][4-méthoxyphénoxy]méthyl]cycloheptanol ;
le 1-[[(diméthylamino)carbonyl][4-méthoxyphénoxy]méthyl]cyclopentanol ;
le 1-[[(diméthylamino)carbonyl][3-trifluorométhylphénoxy]méthyl]cyclohexanol ;
le 1-[[(diméthylamino)carbonyl][4-trifluorométhylphénoxy]méthyl]cyclohexanol ;
le 1-[[(diméthylamino)carbonyl][4-trifluorométhylphénoxy]méthyl]cyclopentanol ;
le 1-[[(diméthylamino)carbonyl][3-méthoxyphénoxy]méthyl]cyclohexanol ;
le 1-[[(diméthylamino)carbonyl][3-méthoxyphénoxy]méthyl]cycloheptanol ou
le 1-[[(diméthylamino)carbonyl][3-méthoxyphénoxy]méthyl]cyclopentanol.

**9.** Procédé de préparation d'un composé de formule I ou d'un sel pharmaceutiquement acceptable de ce composé tel que défini dans la revendication 1, qui consiste :

(i) à réduire un composé de formule A

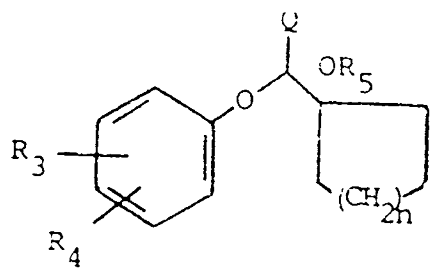

(A)

dans laquelle Q représente $-CONR_1R_2$ ; $-CH=NR_2$ ; $-CH(OH)NR_1R_2$ ; $-CH_2NR_1COR_9$ ; $-CH_2N=CY_1Y_2$ ; $-CH_2NR_1CH(OH)R_8$,

$R_9$ représente un groupe alkoxy, alkyle en $C_1$ à $C_5$ ou l'hydrogène ;

$Y_1$ et $Y_2$ sont choisis chacun entre l'hydrogène et un groupe alkyle de manière que le groupe $=CY_1Y_2$ contienne 1 à 6 atomes de carbone,

$R_8$ est un groupe alkyle en $C_1$ à $C_5$

et $n_1$ $R_1$, $R_2$, $R_3$, $R_4$ et $R_5$ sont tels que définis dans la revendication 1

ou bien

(2) (a) à faire réagir un composé répondant à la formule B

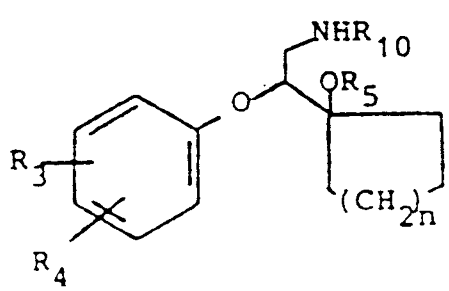

(B)

(dans laquelle $R_{10}$ représente $R_1$ ou un substituant aisément éliminable et $R_3$, $R_4$ et $R_5$ ont les définitions données dans la revendication 1) avec un agent alkylant pour introduire un ou deux groupes alkyle en $C_1$ à $C_6$ (si $R_{10}$ représente H) ou un tel groupe alkyle et, si nécessaire, élimination dudit substituant aisément éliminable ; ou bien

(b) à faire réagir un composé de formule B telle que définie ci-dessus, dans laquelle $R_{10}$ représente $R_1$ avec un composé carbonylique de formule $Y_1Y_2CO$ [dans laquelle $Y_1$ et $Y_2$ sont tels que définis ci-dessus] en présence d'un agent réducteur ; ou bien

(3) (a) à faire réagir un composé de formule $HNR_2R_{10}$ (dans laquelle $R_2$ et $R_1$ sont tels que définis ci-dessus) avec un composé répondant à la formule C

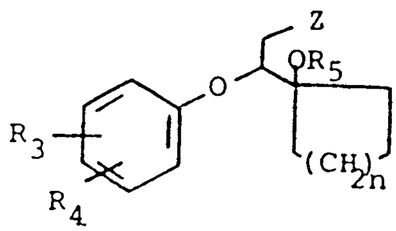

(C)

(dans laquelle Z est un groupe partant et $R_3$, $R_4$ et $R_5$ ont les définitions données pour la formule I) et, si nécessaire, à éliminer le substituant $R_{10}$ ; ou bien
(b) à faire réaqgir un composé de formule $HNR_1R_2$ (dans laquelle $R_1$ et $R_2$ sont tels que définis ci-dessus) avec un aldéhyde de formule D

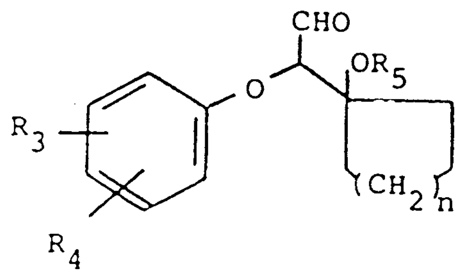

(D)

(dans laquelle $R_3$, $R_4$, $R_5$ et n sont tels que définis dans la revendication 1) en présence d'un agent réducteur, ou bien
(4) à faire réagir un composé de formule I dans laquelle $R_5$ est l'hydrogène avec un dérivé réactif d'un acide alcanoïque en $C_2$ à $C_7$ pour introduire un groupe alcanoyle en $C_2$ à $C_7$ en tant que groupe $R_5$ ; ou bien
(5) à former un composé de formule I ou un sel de ce composé par élimination d'un groupe protecteur pour que $R_3$ et/ou $R_4$ et/ou $OR_5$ représentent un groupe hydroxy, ou bien
(6) à faire réagir un composé de formule I sous la forme de la base libre avec un acide pour former un sel pharmaceutiquement acceptable de ce composé ou à faire réagir un sel d'un composé de formule I avec une base pour obtenir le composé de formule I sous la forme de la base libre.

**10.** Procédé de préparation d'un composé de formule II suivant la revendication 2, qui consiste
(a) à faire réagir un anion de formule E

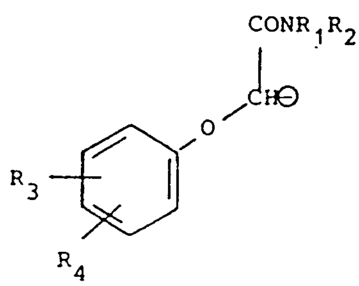

(E)

(dans laquelle $R_1$, $R_2$, $R_3$ et $R_4$ sont tels que définis dans la revendication 1) avec une cycloalcanone de formule F

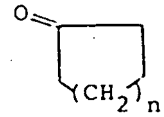

(F)

(dans laquelle n est tel que défini dans la revendication 1) et, le cas échéant, à introduire un groupe alkyle $R_5$ par alkylation ; ou bien

(b) à faire réagir une amine répondant à la formule $HNR_1R_2$ (dans laquelle $R_1$ et $R_2$ sont tels que définis ci-dessus) avec le dérivé halogénure d'acide, un ester actif ou un anhydride d'un acide répondant à la formule (G)

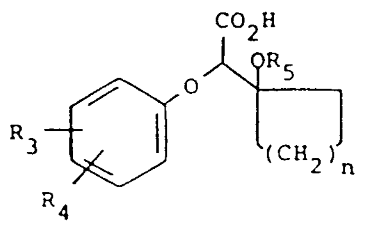

(G)

(dans laquelle $R_3$, $R_4$, $R_5$ et n ont les définitions données dans la revendication 2).

**11.** Composé de formule I suivant l'une quelconque des revendications 1 et 3 à 7, destiné à être utilisé comme substance pharmaceutique.

**12.** Composition pharmaceutique comprenant un composé de formule I ou un sel pharmaceutiquement acceptable de ce composé suivant l'une quelconque des revendications 1 et 3 à 7 et un support acceptable du point de vue pharmaceutique.

**Revendications pour l'Etat contractant suivant : AT**

**1.** Procédé de préparation d'un composé de formule I

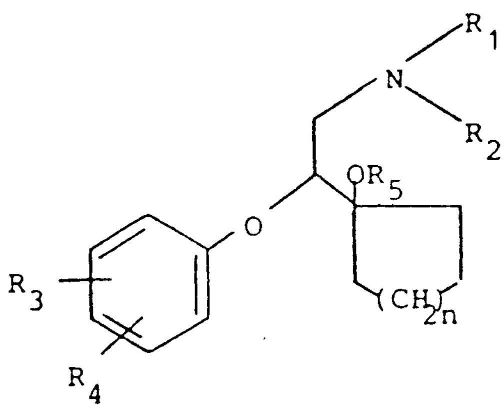

(I)

dans laquelle

| | |
|---|---|
| $R_1$ | est l'hydrogène ou un groupe alkyle ayant 1 à 6 atomes de carbone ; |
| $R_2$ | est un groupe alkyle de 1 à 6 atomes de carbone ; |
| $R_3$ et $R_4$ | représentent indépendamment l'hydrogène, un groupe hydroxyle, alkyle ayant 1 à 6 atomes de carbone, alkoxy ayant 1 à 6 atomes de carbone, alcanoyloxy ayant 2 à 7 atomes de carbone, halogéno ou trifluorométhyle ; |
| $R_5$ | est l'hydrogène, un groupe alkyle ayant 1 à 6 atomes de carbone ou alcanoyle ayant 2 à 7 atomes de carbone ; et |
| n | est l'un des nombres entiers 0, 1, 2 et 3 ; |

ou un sel pharmaceutiquement acceptable de ce composé, qui consiste

(i) à réduire un composé de formule A

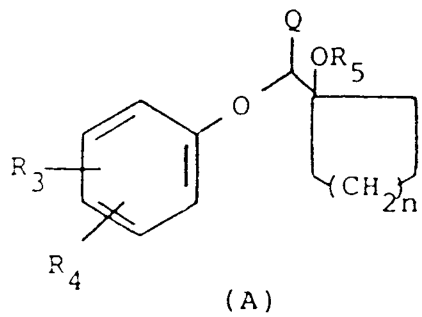

(A)

dans laquelle Q représente $-CONR_1R_2$ ; $-CH=NR_2$ ; $-CH(OH)NR_1R_2$ ; $-CH_2NR_1COR_9$ ; $-CH_2N=CY_1Y_2$ ; $-CH_2NR_1CH(OH)R_8$,

$R_9$ représente un groupe alkoxy, alkyle en $C_1$ à $C_5$ ou l'hydrogène ;

$Y_1$ et $Y_2$ sont choisis chacun entre l'hydrogène et un groupe alkyle de manière que le groupe $=CR_1Y_2$ contienne 1 à 6 atomes de carbone,

$R_8$ est un groupe alkyle en $C_1$ à $C_5$

et n, $R_3$, $R_4$ et $R_5$ sont tels que définis ci-dessus ; ou bien

(ii)(a) à faire réagir un composé répondant à la formule B

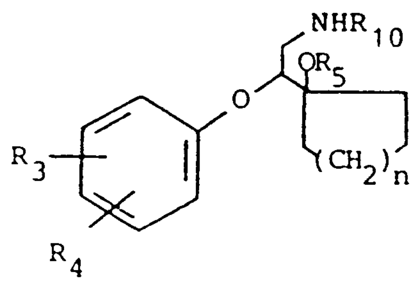

(dans laquelle $R_{10}$ représente $R_1$ ou un substituant aisément éliminable et $R_3$, $R_4$ et $R_5$ ont les définitions données ci-dessus) avec un agent alkylant pour introduire un ou deux groupes alkyle en $C_1$ à $C_6$ (si $R_{10}$ représente H) ou un tel groupe alkyle et, si nécessaire, à éliminer le substituant aisément éliminable ;

ou bien (b) à faire réagir un composé de formule B telle que définie ci-dessus, dans laquelle $R_{10}$ représente $R_1$, avec un composé carbonylique de formule $Y_1Y_2CO$ [dans laquelle $Y_1$ et $Y_2$ sont tels que définis ci-dessus] en présence d'un agent réducteur ; ou bien (iii)(a) à faire réagir un composé de formule $HNR_2R_{10}$ (dans laquelle $R_2$ et $R_{10}$ sont tels que définis ci-dessus) avec un composé répondant à la formule C

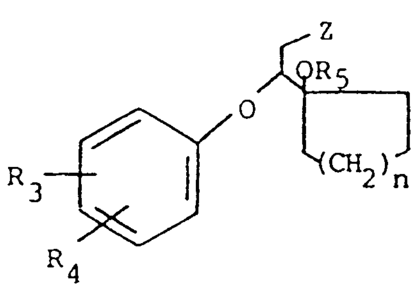

(C)

(dans laquelle Z est un groupe partant et $R_3$, $R_4$ et $R_5$ sont tels que définis pour la formule I) et, si nécessaire, à éliminer le substituant $R_{10}$ ;

ou bien (b) à faire réagir un composé de formule $HNR_1R_2$ (dans laquelle $R_1$ et $R_2$ sont tels que définis ci-dessus) avec un aldéhyde de formule D

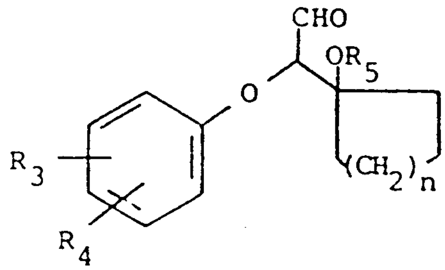

(D)

(dans laquelle $R_3$, $R_4$, $R_5$ et n sont tels que définis ci-dessus)
en présence d'un agent réducteur ;
ou bien
(iv) à faire réagir un composé de formule I dans laquelle $R_5$ est l'hydrogène avec un dérivé réactif d'un acide alcanoïque en $C_2$ à $C_7$ pour introduire un groupe alcanoyle en $C_2$ à $C_7$ en tant que groupe R5 ;
ou bien
(v) à former un composé de formule I ou un sel de ce composé par élimination d'un groupe protecteur de manière que $R_3$ et/ou $R_4$ et/ou $OR_5$ représentent un groupe hydroxy ;
ou bien
(vi) à faire réagir un composé de formule I sous la forme de base libre avec un acide pour former un sel pharmaceutiquement acceptable de ce composé ou à faire réagir un sel d'un composé de formule I avec une base pour obtenir le composé de formule I sous la forme de la base libre.

**2.** Procédé de préparation d'un composé de formule II

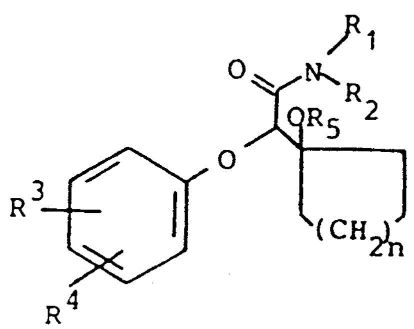

(II)

dans laquelle n, $R_1$, $R_2$, $R_3$ et $R_4$ sont tels que définis dans la revendication 1 à propos de la formule I et $R_5$ est l'hydrogène ou un groupe alkyle ayant 1 à 6 atomes de carbone, qui consiste
(a) à faire réagir un anion de formule E

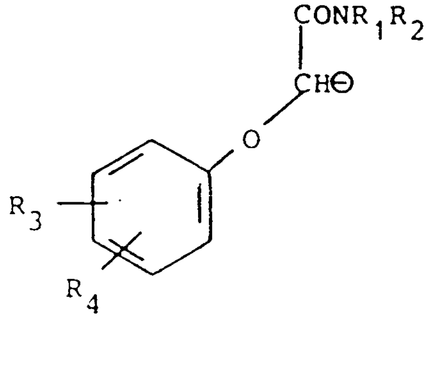

(E)

(dans laquelle $R_1$, $R_2$, $R_3$ et $R_4$ sont tels que définis dans la revendication 1), avec une cycloalcano-

ne de formule F

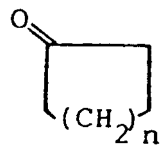

(F)

(dans laquelle n est tel que défini dans la revendication 1), et, le cas échéant, à introduire un groupe alkyle $R_5$ par alkylatioin ; ou bien
(b) à faire réagir une amine répondant à la formule $HNR_1R_2$ (dans laquelle $R_1$ et $R_2$ sont tels que définis ci-dessus) avec le dérivé halogénure d'acide, un ester actif ou l'anhydride d'un acide répondant à la formule (G)

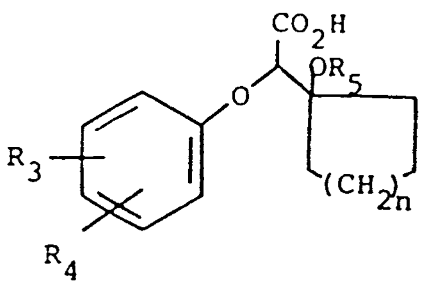

(G)

dans laquelle n, $R_3$, $R_4$ et $R_5$ sont tels que définis ci-dessus.

**3.** Procédé suivant la revendication 1 ou la revendication 2, dans lequel $R_1$ et/ou $R_2$ représentent un groupe alkyle ayant 1 à 3 atomes de carbone.

**4.** Procédé suivant l'une quelconque des revendications 1 à 3, dans lequel $R_3$ est l'hydrogène, un groupe alkoxy ayant 1 à 3 atomes de carbone, chloro, bromo ou trifluorométhyle.

**5.** Procédé suivant l'une quelconque des revendications 1 à 4, dans lequel $R_4$ est un groupe alkyle ayant 1 à 3 atomes de carbone, alkoxy ayant 1 à 3 atomes de carbone, chloro, bromo ou trifluorométhyle.

**6.** Procédé suivant l'une quelconque des revendications 1 à 5, dans lequel $R_3$ et $R_4$ sont en positions méta ou para.

**7.** Procédé suivant la revendication 1, dans lequel le composé préparé est
le 1-[1-(4-chlorophénoxy)-2-diméthylamino)-éthyl]-cyclohexanol ;
le 1-[1-(2,4-dichlorophénoxy)-2-(diméthylamino)éthyl]cyclohexanol ;
le 1-[1-(2,4-dichlorophénoxy)-2-(diméthylamino)éthyl]cyclopentanol ;
le 1-[2-(diméthylamino)-1-(4-méthoxyphénoxy)éthyl]cyclohexanol ;
le 1-[2-(diméthylamino)-1-(4-méthoxyphénoxy)éthyl]cycloheptanol ;
le 1-[2-(diméthylamino)-1-(4-méthoxyphénoxy)éthyl]cyclopentanol ;
le 1-[2-(diméthylamino)-1-(3-trifluorométhylphénoxy)éthyl]cyclohexanol ;
le 1-[2-(diméthylamino)-1-(4-trifluorométhylphénoxy)éthyl]cyclohexanol ;
le 1-[2-(diméthylamino)-1-(4-trifluorométhylphénoxy)éthyl]cycloheptanol ;
le 1-[2-(diméthylamino)-1-(3-méthoxyphénoxy)éthyl]cyclohexanol ;
le 1-[2-(diméthylamino)-1-(3-méthoxyphénoxy)éthyl)cycloheptanol ; ou
le 1-[2-(diméthylamino)-1-(3-méthoxyphénoxy)éthyl]cyclopentanol ; ou un sel pharmaceutiquement acceptable de ce composé.

**8.** Procédé suivant la revendication 2, dans lequel le composé préparé est
le 1-[(4-chlorophénoxy)((diméthylamino)carbonyl)méthyl)cyclohexanol ;

le 1-[(2,4-dichlorophénoxy)((diméthylamino)carbonyl)méthyl]cyclohexanol ;
le [(2,4-dichlorophénoxy)((diméthylamino)carbonyl)méthyl)cyclopentanol ;
le 1-[[(diméthylamino)carbonyl][4-méthoxyphénoxy]méthyl]cyclohexanol ;
le 1-[[(diméthylamino)carbonyl][4-méthoxyphénoxy]méthyl]cycloheptanol ;
le 1-[[(diméthylamino)carbonyl][4-méthoxyphénoxy]méthyl]cyclopentanol ;
le 1-[[(diméthylamino)carbonyl][3-trifluorométhylphénoxy]méthyl]cyclohexanol ; ou
le 1-[[(diméthylamino)carbonyl][4-trifluorométhylphénoxy]méthyl]cyclohexanol ;
le 1-[[(diméthylamino)carbonyl][4-trifluorométhylphénoxy]méthyl]cycloheptanol ;
le 1-[[(diméthylamino)carbonyl][3-méthoxyphénoxy]méthyl]cyclohexanol ;
le 1-[[(diméthylamino)carbonyl][3-méthoxyphénoxy]méthyl]cycloheptanol ; ou
le 1-[[(diméthylamino)carbonyl][3-méthoxyphénoxy]méthyl]cyclopentanol.

**9.** Procédé de préparation d'une composition pharmaceutique, qui consiste à mettre un composé de formule I ou un sel pharmaceutiquement acceptable de ce composé tel que défini dans la revendication 1 en association avec un support acceptable du point de vue pharmaceutique.

**10.** Procédé de préparation d'une composition pharmaceutique, qui consiste à mettre un composé de formule I préparé conformément à l'une quelconque des revendications 1 et 3 à 7 en association avec un support pharmaceutiquement acceptable.

**Patentansprüche**
**Patentansprüche für folgende Vertragsstaaten : BE, CH, DE, FR, IT, LI, LU, NL, SE**

**1.** Verbindung der Formel (I)

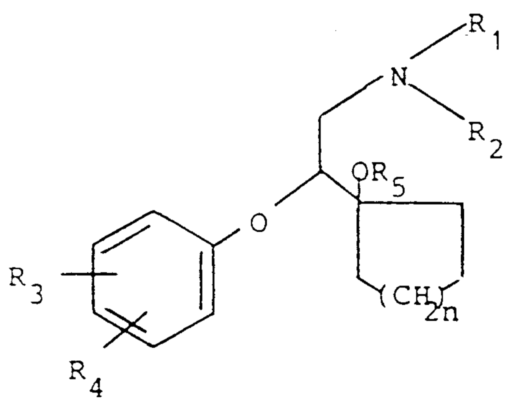

(I),

worin $R_1$ Wasserstoff oder Alkyl mit 1 bis 6 Kohlenstoffatomen bedeutet; $R_2$ Alkyl mit 1 bis 6 Kohlenstoffatomen darstellt; $R_3$ und $R_4$ unabhängig Wasserstoff, Hydroxyl, Alkyl mit 1 bis 6 Kohlenstoffatomen, Alkoxy mit 1 bis 6 Kohlenstoffatomen, Alkanoyloxy mit 2 bis 7 Kohlenstoffatomen, Halogen oder Trifluormethyl sind; $R_5$ Wasserstoff, Alkyl mit 1 bis 6 Kohlenstoffatomen oder Alkanoyl mit 2 bis 7 Kohlenstoffatomen bedeutet; und n eine der ganzen Zahlen 0, 1, 2 oder 3 ist; oder ein pharmazeutisch annehmbares Salz hievon.

**2.** Verbindung der Formel (II)

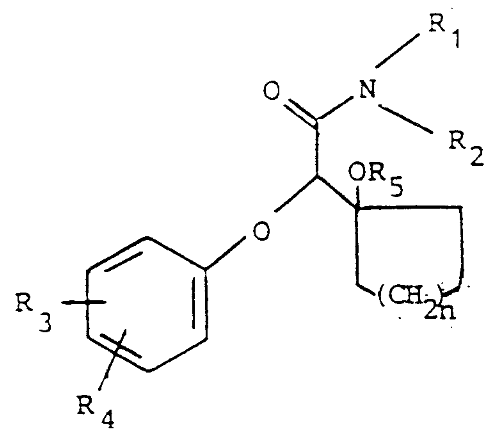

(II),

worin n, $R_1$, $R_2$, $R_3$ und $R_4$ wie in Anspruch 1 definiert sind und $R_5$ Wasserstoff oder Alkyl mit 1 bis 6 Kohlenstoffatomen bedeutet.

3. Verbindung der Formel (I) oder (II) nach Anspruch 1 oder 2, worin $R_1$ und/oder $R_2$ Alkyl mit 1 bis 3 Kohlenstoffatomen ist/sind.

4. Verbindung der Formel (I) oder (II) nach einem der Ansprüche 1 bis 3, worin $R_3$ Wasserstoff, Alkoxy mit 1 bis 3 Kohlenstoffatomen, Chlor, Brom oder Trifluormethyl bedeutet.

5. Verbindung der Formel (I) oder (II) nach einem der Ansprüche 1 bis 4, worin $R_4$ Alkyl mit 1 bis 3 Kohlenstoffatomen, Alkoxy mit 1 bis 3 Kohlenstoffatomen, Chlor, Brom oder Trifluormethyl darstellt.

6. Verbindung der Formel (I) oder (II) nach einem der Ansprüche 1 bis 5, worin $R_3$ und $R_4$ in m- oder p-Stellungen vorliegen.

7. Verbindung nach Anspruch 1, die
1-[1-(4-Chlorphenoxy)-2-(dimethylamino)-ethyl]-cyclohexanol;
1-[1-(2,4-Dichlorphenoxy)-2-(dimethylamino)-ethyl]-cyclohexanol;
1-[1-(2,4-Dichlorphenoxy)-2-(dimethylamino)-ethyl]-cyclopentanol;
1-[2-(Dimethylamino)-1-(4-methoxyphenoxy)-ethyl]-cyclohexanol;
1-[2-(Dimethylamino)-1-(4-methoxyphenoxy)-ethyl]-cycloheptanol;
1-12-(Dimethylamino)-1-(4-methoxyphenoxy)-ethyl]-cyclopentanol;
1-[2-(Dimethylamino)-1-(3-trifluormethylphenoxy)-ethyl]-cyclohexanol;
1-[2-(Dimethylamino)-1-(4-trifluormethylphenoxy)-ethyl]-cyclohexanol;
1-[2-(Dimethylamino)-1-(4-trifluormethylphenoxy)-ethyl]-cycloheptanol;
1-[2-(Dimethylamino)-1-(3-methoxyphenoxy)-ethyl]-cyclohexanol;
1-[2-(Dimethylamino)-1-(3-methoxyphenoxy)-ethyl]-cycloheptanol
oder
1-[2-(Dimethylamino)-1-(3-methoxyphenoxy)-ethyl]-cyclopentanol oder ein pharmazeutisch annehmbares Salz hievon ist.

8. Verbindung nach Anspruch 2, die
1-[(4-Chlorphenoxy)-((dimethylamino)-carbonyl)-methyl]-cyclohexanol;
1-[(2,4-Dichlorphenoxy)-((dimethylamino)-carbonyl)-methyl]-cyclohexanol;
1-[(2,4-Dichlorphenoxy)-((dimethylamino)-carbonyl)-methyl]-cyclopentanol;
1-[[(Dimethylamino)-carbonyl]-[4-methoxyphenoxy]-methyl]-cyclohexanol;
1-[[(Dimethylamino)-carbonyl]-[4-methoxyphenoxy]-methyl]-cycloheptanol;
1-[[(Dimethylamino)-carbonyl]-[4-methoxyphenoxy]-methyl]-cyclopentanol;
1-[[(Dimethylamino)-carbonyl]-[3-trifluormethylphenoxy]-methyl]-cyclohexanol;
1-[[(Dimethylamino)-carbonyl]-[4-trifluormethylphenoxy]-methyl]-cyclohexanol;
1-[[(Dimethylamino)-carbonyl]-[4-trifluormethylphenoxy]-methyl]-cycloheptanol;
1-[[(Dimethylamino)-carbonyl]-[3-methoxyphenoxy]-methyl]-cyclohexanol;
1-[[(Dimethylamino)-carbonyl]-[3-methoxyphenoxy]-methyl]-cycloheptanol oder

1-[[(Dimethylamino)-carbonyl]-[3-methoxyphenoxy]-methyl]-cyclopentanol ist.

**9.** Verfahren zur Herstellung einer Verbindung der Formel (I) oder eines pharmazeutisch annehmbaren Salzes hievon wie in Anspruch 1 definiert, welches Verfahren

(1) Reduzieren einer Verbindung der Formel (A)

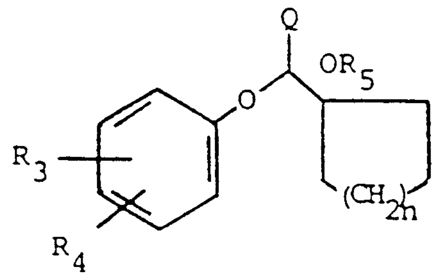

(A),

worin Q die Bedeutung $-CONR_1R_2$; $-CH=NR_2$; $-CH(OH)NR_1R_2$; $-CH_2NR_1COR_9$; $-CH_2N=CY_1Y_2$; $-CH_2NR_1CH(OH)R_8$ hat, $R_9$ Alkoxy, $C_1$-$C_5$-Alkyl oder Wasserstoff ist; $Y_1$ und $Y_2$ jeweils aus Wasserstoff und Alkyl ausgewählt sind, so daß die Gruppe $=CY_1Y_2$ 1 bis 6 Kohlenstoffatome enthält, $R_8$ $C_1$-$C_5$-Alkyl darstellt und n, $R_1$, $R_2$, $R_3$, $R_4$ und $R_5$ wie in Anspruch 1 definiert sind, oder

(2) (a) Umsetzen einer Verbindung der Formel (B)

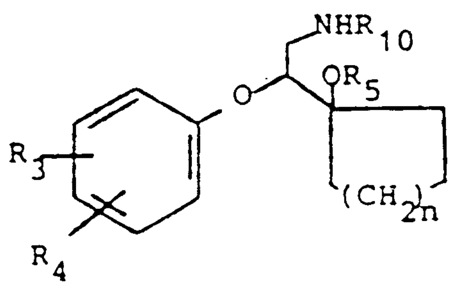

(B),

worin $R_{10}$ $R_1$ oder einen leicht abspaltbaren Substituenten bedeutet, und $R_3$, $R_4$ und $R_5$ wie in Anspruch 1 definiert sind, mit einem Alkylierungsmittel, wobei eine oder zwei $C_1$-$C_6$-Alkylgruppen (wenn $R_{10}$ die Bedeutung H hat) oder eine derartige Alkylgruppe eingeführt werden, und, wenn notwendig, Abspalten des leicht abspaltbaren Substituenten; oder

(b) Umsetzen einer Verbindung der Formel (B) wie oben definiert, worin $R_{10}$ $R_1$ bedeutet, mit einer Carbonylverbindung der Formel $Y_1Y_2CO$ (worin $Y_1$ und $Y_2$ wie oben definiert sind) in Anwesenheit eines Reduktionsmittels; oder

(3) (a) Umsetzen einer Verbindung der Formel $HNR_2R_{10}$ (worin $R_2$ und $R_{10}$ wie oben definiert sind) mit einer Verbindung der Formel (C)

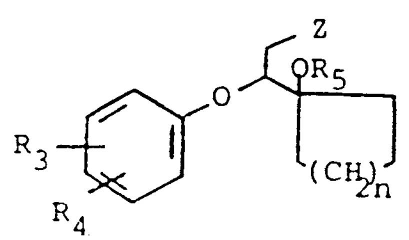

(C),

worin Z eine Abgangsgruppe ist und $R_3$, $R_4$ und $R_5$ wie in Formel (I) definiert sind, und, wenn notwendig, Abspalten des Substituenten $R_{10}$; oder

(b) Umsetzen einer Verbindung der Formel $HNR_1R_2$ (worin $R_1$ und $R_2$ wie oben definiert sind) mit einem Aldehyd der Formel (D)

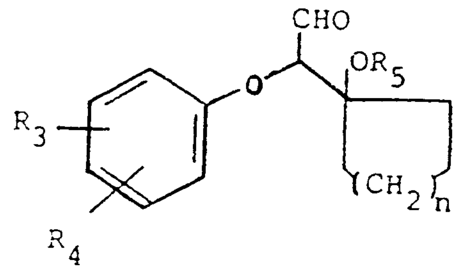

(D),

worin $R_3$, $R_4$, $R_5$ und n wie in Anspruch 1 definiert sind, in Anwesenheit eines Reduktionsmittels oder

(4) Umsetzen einer Verbindung der Formel (I), worin $R_5$ Wasserstoff ist, mit einem reaktiven Derivat einer $C_2$-$C_7$-Alkansäure, wobei $C_2$-$C_7$-Alkanoyl als $R_5$ eingeführt wird; oder

(5) Bilden einer Verbindung der Formel (I) oder eines Salzes hievon durch Entfernen einer Schutzgruppe, wobei Hydroxy als $R_3$ und/oder $R_4$ und/oder $OR_5$ erhalten wird, oder

(6) Umsetzen einer Verbindung der Formel (I) in freier Basenform mit einer Säure, wobei ein pharmazeutisch annehmbares Salz hievon gebildet wird, oder Umsetzen eines Salzes einer Verbindung der Formel (I) mit einer Base, wobei die Verbindung der Formel (I) in freier Basenform erhalten wird, umfaßt.

**10.** Verfahren zur Herstellung einer Verbindung der Formel (II) wie in Anspruch 2 definiert, welches Verfahren

(a) Umsetzen eines Anions der Formel (E)

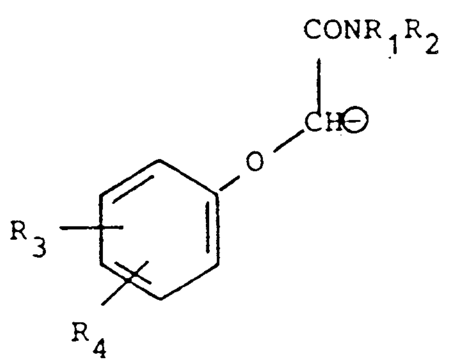

(E),

worin $R_1$, $R_2$, $R_3$ und $R_4$ wie in Anspruch 1 definiert sind, mit einem Cycloalkanon der Formel (F)

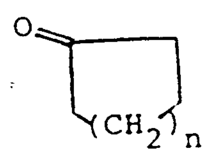

(F),

worin n wie in Anspruch 1 definiert ist, und, wenn gewünscht, Einführen einer $R_5$-Alkylgruppe durch Alkylieren; oder

(b) Umsetzen eines Amins der Formel $HNR_1R_2$ (worin $R_1$ und $R_2$ wie oben definiert sind) mit dem Säurehalogenidderivat, aktiven Ester oder Anhydrid einer Säure der Formel (G),

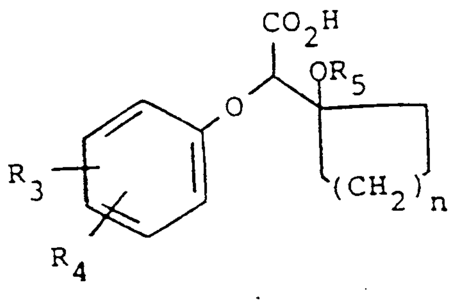

(G),

worin $R_3$, $R_4$, $R_5$ und n wie in Anspruch 2 definiert sind, umfaßt.

11. Verbindung der Formel (I) nach einem der Ansprüche 1 und 3 bis 7 zur Verwendung als Pharmazeutikum.

12. Pharmazeutische Zusammensetzung umfassend eine Verbindung der Formel (I) oder ein pharmazeutisch annehmbares Salz hievon nach einem der Ansprüche 1 und 3 bis 7 und einen pharmazeutisch annehmbaren Träger.

**Patentansprüche für folgende Vertragsstaat : AT**

1. Verfahren zur Herstellung einer Verbindung der Formel (I)

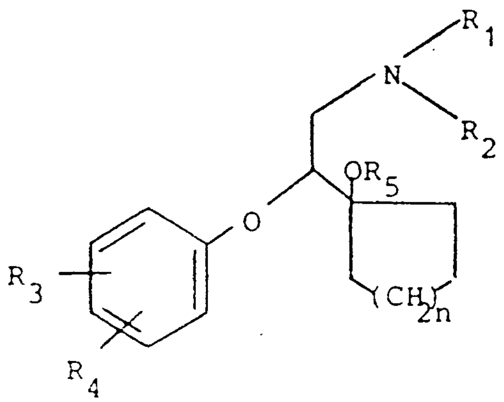

(I),

worin $R_1$ Wasserstoff oder Alkyl mit 1 bis 6 Kohlenstoffatomen bedeutet; R Alkyl mit 1 bis 6 Kohlenstoffatomen darstellt; $R_3$ und $R_4$ unabhängig Wasserstoff, Hydroxyl, Alkyl mit 1 bis 6 Kohlenstoffatomen, Alkoxy mit 1 bis 6 Kohlenstoffatomen, Alkanoyloxy mit 2 bis 7 Kohlenstoffatomen, Halogen oder Trifluormethyl sind; $R_5$ Wasserstoff, Alkyl mit 1 bis 6 Kohlenstoffatomen oder Alkanoyl mit 2 bis 7 Kohlenstoffatomen bedeutet; und n eine der ganzen Zahlen 0, 1, 2 oder 3 ist; oder eines pharmazeutisch annehmbaren Salzes hievon, welches Verfahren

(i) Reduzieren einer Verbindung der Formel (A)

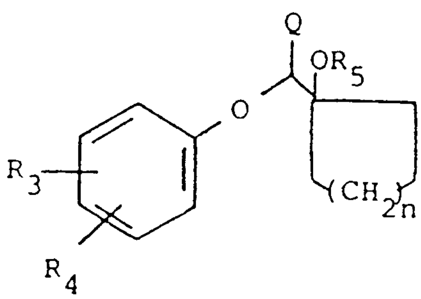

(A),

worin Q die Bedeutung $-CONR_1R_2$; $-CH=NR_2$; $-CH(OH)NR_1R_2$; $-CH_2NR_1COR_9$; $-CH_2N=CY_1Y_2$;

$-CH_2NR_1CH(OH)R_8$ hat, $R_9$ Alkoxy, $C_1$-$C_5$-Alkyl oder Wasserstoff ist; $Y_1$ und $Y_2$ jeweils aus Wasserstoff und Alkyl ausgewählt sind, so daß die Gruppe $=CY_1Y_2$ 1 bis 6 Kohlenstoffatome enthält, $R_8$ $C_1$-$C_5$-Alkyl darstellt und n, $R_3$, $R_4$ und $R_5$ wie oben definiert sind, oder

(ii) (a) Umsetzen einer Verbindung der Formel (B)

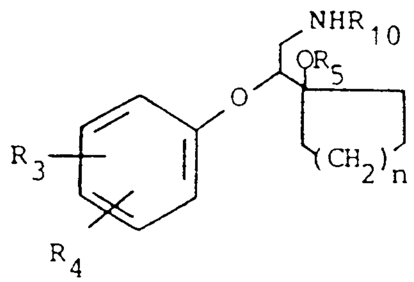

(B),

worin $R_{10}$ $R_1$ oder einen leicht abspaltbaren Substituenten bedeutet, und $R_3$, $R_4$ und $R_5$ wie oben definiert sind, mit einem Alkylierungsmittel, wobei eine oder zwei $C_1$-$C_6$-Alkylgruppen (wenn $R_{10}$ die Bedeutung H hat) oder eine derartige Alkylgruppe eingeführt werden, und, wenn notwendig, Abspalten des leicht abspaltbaren Substituenten; oder

(b) Umsetzen einer Verbindung der Formel (B) wie oben definiert, worin $R_{10}$ $R_1$ bedeutet, mit einer Carbonylverbindung der Formel $Y_1Y_2CO$ (worin $Y_1$ und $Y_2$ wie oben definiert sind) in Anwesenheit eines Reduktionsmittels; oder

(iii)(a) Umsetzen einer Verbindung der Formel $HNR_2R_{10}$ (worin $R_2$ und $R_{10}$ wie oben definiert sind) mit einer Verbindung der Formel (C)

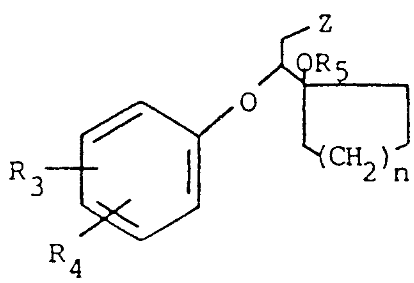

(C),

worin Z eine Abgangsgruppe ist und $R_3$, $R_4$ und $R_5$ wie in Formel (I) definiert sind, und, wenn notwendig, Abspalten des Substituenten $R_{10}$; oder

(b) Umsetzen einer Verbindung der Formel $HNR_1R_2$ (worin $R_1$ und $R_2$ wie oben definiert sind) mit einem Aldehyd der Formel (D)

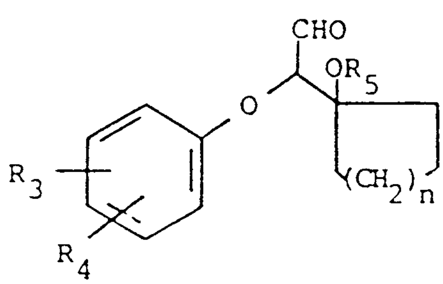

(D),

worin $R_3$, $R_4$, $R_5$ und n wie oben definiert sind, in Anwesenheit eines Reduktionsmittels; oder

(iv) Umsetzen einer Verbindung der Formel (I), worin $R_5$ Wasserstoff ist, mit einem reaktiven Derivat einer $C_2$-$C_7$-Alkansäure, wobei $C_2$-$C_7$-Alkanoyl als $R_5$ eingeführt wird; oder

(v) Bilden einer Verbindung der Formel (I) oder eines Salzes hievon durch Entfernen einer Schutzgruppe, wobei Hydroxy als $R_3$ und/oder $R_4$ und/oder $OR_5$ erhalten wird, oder

(vi) Umsetzen einer Verbindung der Formel (I) in freier Basenform mit einer Säure, wobei ein pharmazeutisch annehmbares Salz hievon gebildet wird, oder Umsetzen eines Salzes einer Verbindung der Formel (I) mit einer Base, wobei die Verbindung der Formel (I) in freier Basenform erhalten wird, umfaßt.

**2.** Verfahren zur Herstellung einer Verbindung der Formel (II)

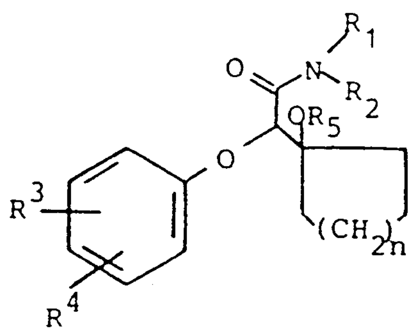

(II),

worin n, $R_1$, $R_2$, $R_3$ und $R_4$ wie in Anspruch 1 in Verbindung mit Formel (I) definiert sind und $R_5$ Wasserstoff oder Alkyl mit 1 bis 6 Kohlenstoffatomen bedeutet, welches Verfahren

(a) Umsetzen eines Anions der Formel (E)

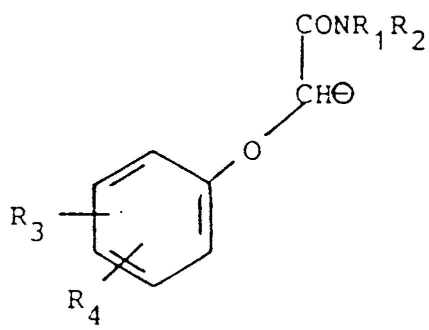

(E),

worin $R_1$, $R_2$, $R_3$ und $R_4$ wie in Anspruch 1 definiert sind, mit einem Cycloalkanon der Formel (F)

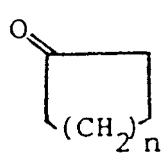

(F),

worin n wie in Anspruch 1 definiert ist, und, wenn gewünscht, Einführen einer $R_5$-Alkylgruppe durch Alkylieren; oder

(b) Umsetzen eines Amins der Formel $HNR_1R_2$ (worin $R_1$ und $R_2$ wie oben definiert sind) mit dem Säurehalogenidderivat, aktiven Ester oder Anhydrid einer Säure der Formel (G),

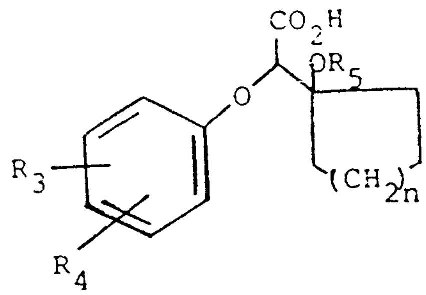

(G),

worin n, $R_3$, $R_4$ und $R_5$ wie oben definiert sind, umfaßt.

3. Verfahren nach Anspruch 1 oder 2, worin $R_1$ und/oder $R_2$ Alkyl mit 1 bis 3 Kohlenstoffatomen ist/sind.

4. Verfahren nach einem der Ansprüche 1 bis 3, worin $R_3$ Wasserstoff, Alkoxy mit 1 bis 3 Kohlenstoffatomen, Chlor, Brom oder Trifluormethyl bedeutet.

5. Verfahren nach einem der Ansprüche 1 bis 4, worin $R_4$ Alkyl mit 1 bis 3 Kohlenstoffatomen, Alkoxy mit 1 bis 3 Kohlenstoffatomen, Chlor, Brom oder Trifluormethyl darstellt.

6. Verfahren nach einem der Ansprüche 1 bis 5, worin $R_3$ und $R_4$ in m- oder p-Stellungen vorliegen.

7. Verfahren nach Anspruch 1, worin die hergestellte Verbindung
1-[1-(4-Chlorphenoxy)-2-(dimethylamino)-ethyl]-cyclohexanol;
1-[1-(2,4-Dichlorphenoxy)-2-(dimethylamino)-ethyl]-cyclohexanol;
1-[1-(2,4-Dichlorphenoxy)-2-(dimethylamino)-ethyl]-cyclopentanol;
1-[2-(Dimethylamino)-1-(4-methoxyphenoxy)-ethyl]-cyclohexanol;
1-[2-(Dimethylamino)-1-(4-methoxyphenoxy)-ethyl]-cycloheptanol;
1-[2-(Dimethylamino)-1-(4-methoxyphenoxy)-ethyl]-cyclopentanol;
1-[2-(Dimethylamino)-1-(3-trifluormethylphenoxy)-ethyl]-cyclohexanol;
1-[2-(Dimethylamino)-1-(4-trifluormethylphenoxy)-ethyl]-cyclohexanol;
1-[2-(Dimethylamino)-1-(4-trifluormethylphenoxy)-ethyl]-cycloheptanol;
1-[2-(Dimethylamino)-1-(3-methoxyphenoxy)-ethyl]-cyclohexanol;
1-[2-(Dimethylamino)-1-(3-methoxyphenoxy)-ethyl]-cycloheptanol oder
1-[2-(Dimethylamino)-1-(3-methoxyphenoxy)-ethyl]-cyclopentanol oder ein pharmazeutisch annehmbares Salz hievon ist.

8. Verfahren nach Anspruch 2, worin die hergestellte Verbindung
1-[(4-Chlorphenoxy)-((dimethylamino)-carbonyl)-methyl]-cyclohexanol;
1-[(2,4-Dichlorphenoxy)-((dimethylamino)-carbonyl)-methyl]-cyclohexanol;
1-[(2,4-Dichlorphenoxy)-((dimethylamino)-carbonyl)-methyl]-cyclopentanol;
1-[[(Dimethylamino)-carbonyl]-[4-methoxyphenoxy]-methyl]-cyclohexanol;
1-[[(Dimethylamino)-carbonyl]-[4-methoxyphenoxy]-methyl]-cycloheptanol;
1-[[(Dimethylamino)-carbonyl]-[4-methoxyphenoxy]-methyl]-cyclopentanol;
1-[[(Dimethylamino)-carbonyl]-[3-trifluormethylphenoxy]-methyl]-cyclohexanol; oder
1-[[(Dimethylamino)-carbonyl]-[4-trifluormethylphenoxy]-methyl]-cyclohexanol;
1-[[(Dimethylamino)-carbonyl]-[4-trifluormethylphenoxy]-methyl]-cycloheptanol;
1-[[(Dimethylamino)-carbonyl]-[3-methoxyphenoxy]-methyl]-cyclohexanol;
1-[[(Dimethylamino)-carbonyl]-[3-methoxyphenoxy]-methyl]-cycloheptanol oder
1-[[(Dimethylamino)-carbonyl]-[3-methoxyphenoxy]-methyl]-cyclopentanol ist.

9. Verfahren zur Herstellung einer pharmazeutischen Zusammensetzung, welches Verfahren das Vereinigen einer Verbindung der Formel (I) oder eines pharmazeutisch annehmbaren Salzes hievon wie in Anspruch 1 definiert mit einem pharmazeutisch annehmbaren Träger umfaßt.

10. Verfahren zur Herstellung einer pharmazeutischen Zusammensetzung, welches Verfahren das Vereinigen einer Verbindung der Formel (I), hergestellt nach einem der Ansprüche 1 und 3 bis 7, mit einem pharmazeutisch annehmbaren Träger umfaßt.